# FASCICULE DE BREVET EUROPEEN

(11) **EP 3 743 059 B1**
(45) Date de publication et mention de la délivrance du brevet: **11.01.2023**
(21) Numéro de dépôt: 19701525.8
(22) Date de dépôt: 25.01.2019
(51) Int. Cl.: A61K 31/216, A61K 31/265, A61K 31/381, A61P 25/04, A61P 29/00, A61P 25/06

(54) **DÉRIVÉS AMINOACIDES CONTENANT UN GROUPEMENT DISULFANYLE SOUS FORME D'UN INHIBITEUR DE NEP ET D'APN POUR LA PRÉVENTION ET LE TRAITEMENT DES DOULEURS RELATIVES AU NERF TRIJUMEAU**
AMINOSÄUREDERIVATE MIT EINER DISULFANYL-GRUPPE IN FORM EINES NEP- UND APN-INHIBITORS ZUR VORBEUGUNG UND BEHANDLUNG VON TRIGEMINUSNEURALGIE
AMINO ACID DERIVATIVES CONTAINING A DISULFANYL GROUP IN THE FORM OF AN NEP AND APN INHIBITOR FOR THE PREVENTION AND TREATMENT OF TRIGEMINAL NERVE PAIN

(30) Priorité: 26.01.2018 FR 1850630
(43) Date de publication de la demande: 02.12.2020
(73) Titulaire: Pharmaleads, 75013 Paris (FR)
(72) Inventeur: PORAS, Hervé, 78450 VILLEPREUX (FR); OUIMET, Tanja, 75012 PARIS (FR); FOURNIE ZALUSKI, Marie Claude, 75011 PARIS (FR); ROQUES, Bernard, 75014 PARIS (FR); WURM, Michel, 63130 ROYAT (FR)
(74) Mandataire: Regimbeau
(86) Numéro de dépôt international: PCT/EP2019/051904
(87) Numéro de publication internationale: WO 2019/145507

(56) Documents cités:
- WO-A1-2007/048787
- FR-A1- 2 997 081
- US-A1- 2009 012 153
- "Pharmaleads to Present PL265 for Ocular Pain Resultsat the ARVO 2017 Annual Meeting in Baltimore", Pharmaleads.com , 5 mai 2017 (2017-05-05), XP002785098, Extrait de l'Internet: URL:http://www.pharmaleads.com/wp-content/ uploads/2017/05/Pharmaleads-ARVO-2017-5.05 .17-FINAL.pdf [extrait le 2018-09-25]
- THIBAULT K ET AL: "Antinociceptive and anti-allodynic effects of oral PL37, a complete inhibitor of enkephalin-catabolizing enzymes, in a rat model of peripheral neuropathic pain induced by vincristine", EUROPEAN JOURNAL OF PHARMACOLOGY, ELSEVIER SCIENCE, NL, vol. 600, no. 1-3, 14 décembre 2008 (2008-12-14), pages 71-77, XP025672086, ISSN: 0014-2999, DOI: 10.1016/J.EJPHAR.2008.10.004 [extrait le 2008-10-10]
- HERVÉ PORAS ET AL: "New Orally Active Dual Enkephalinase Inhibitors (DENKIs) for Central and Peripheral Pain Treatment", JOURNAL OF MEDICINAL CHEMISTRY, vol. 57, no. 13, 24 juin 2014 (2014-06-24) , pages 5748-5763, XP055510116, ISSN: 0022-2623, DOI: 10.1021/jm500602h
- MEUNIER A ET AL: "Attenuation of pain-related behavior in a rat model of trigeminal neuropathic pain by viral-driven enkephalin overproduction in trigeminal ganglion neurons", MOLECULAR THERAPY : THE JOURNAL OF THE AMERICAN SOCIETY OF GENE THERAPY, ACADEMIC PRESS ; NATURE PUBLISHING GROUP, US, vol. 11, no. 4, 1 avril 2005 (2005-04-01), pages 608-616, XP004785011, ISSN: 1525-0016, DOI: 10.1016/J.YMTHE.2004.12.011
- PORAS HERVÉ ET AL: "Modulation of disulfide dual ENKephalinase inhibitors (DENKIs) activity by a transient N-protection for pain alleviation by oral route", EUROPEAN JOURNAL OF MEDICINAL CHEMISTRY, EDITIONS SCIENTIFIQUE ELSEVIER, PARIS, FR, vol. 102, 17 juillet 2015 (2015-07-17), pages 58-67, XP029267386, ISSN: 0223-5234, DOI: 10.1016/J.EJMECH.2015.07.027

## Description

La présente invention se rapporte à des composés de type disulfure mixte comprenant un inhibiteur de NEP et un inhibiteur d'APN (de part et d'autre d'un pont disulfure) pour leur utilisation pour la prévention et/ou le traitement des douleurs relatives au nerf trijumeau.

### ARRIERE-PLAN TECHNOLOGIQUE

### Le nerf trijumeau

La douleur est une réponse nociceptive liée à des stimulations locales dans le corps. La perception de celle-ci au niveau du système nerveux central nécessite la transmission de stimuli douloureux par les fibres nerveuses périphériques. Lors d'une stimulation au niveau des tissus, qu'elle soit thermique, mécanique ou chimique, des signaux électrochimiques sont transmis depuis les terminaisons sensorielles nerveuses vers la colonne vertébrale, et ensuite vers le cerveau où la douleur est ressentie.

Il existe différents types de douleurs, d'origines très variées dont les traitements sont radicalement différents selon le type des douleurs et leur étiologie.

Le système trigéminé est par exemple impliqué dans les phénomènes nociceptifs provenant des zones orale, faciale et crânienne, mais avec des caractéristiques de nociception toutes particulières, ce qui a une incidence sur le traitement. En effet, les structures cranio-faciales telles que la cornée, les méninges et la pulpe dentaire donnent lieu principalement à des sensations de douleur chez l'homme, mais les plus fréquemment rencontrées, comme les douleurs dentaires, sont souvent difficiles à localiser, et certaines céphalées peuvent se produire même en l'absence de toute cause externe identifiable.

Le nerf trijumeau, cinquième paire de nerfs crâniens est constitué de trois branches : les divisions ophtalmique (V1), maxillaire (V2) et mandibulaire (V3). Chaque division innerve l'un des trois dermatomes du visage ainsi que les tissus muqueux, vasculaires, musculaires et méningés sous-jacents. Chez tous les mammifères, la branche ophtalmique, uniquement sensitive, innerve la cornée, la conjonctive voisine, la pointe du nez, la muqueuse intranasale, la paupière supérieure et la peau supra-orbitaire, une partie des méninges (dure-mère et vaisseaux sanguins) ainsi que la peau du front et du cuir chevelu. La division maxillaire, elle aussi uniquement sensitive, innerve la peau infra-orbitale et post-orbitale, la lèvre supérieure, l'aile du nez, la muqueuse maxillaire intra-orale et les dents de la mâchoire supérieure. La branche mandibulaire, à la fois sensitive et motrice, innerve l'articulation temporo-mandibulaire, la peau des lèvres inférieures, la muqueuse mandibulaire intra-orale, les dents de la mâchoire inférieure et la partie antérieure de la langue. La transmission de la douleur aiguë implique l'activation de différents groupes de récepteurs sensoriels sur les fibres périphériques Aδ et C ainsi que les nocicepteurs, qui répondent aux stimuli mécaniques, thermiques et chimiques nocifs.

Les voies sensorielles qui transmettent les stimuli nociceptifs cranio-faciaux à des étages plus élevés du cerveau proviennent de nocicepteurs ganglionnaires trigéminés et de leurs noyaux associés dans le complexe sensoriel du tronc cérébral du trijumeau et de la moelle épinière cervicale supérieure. Ces structures collectent simultanément des activités de base provenant de nombreuses sources qui ne sont pas seulement pertinentes pour la douleur, mais qui peuvent également jouer un rôle dans la transmission continue d'informations cruciales pour maintenir l'intégrité des régions cranio-faciales. Certaines caractéristiques particulières de la nociception du trijumeau peuvent donc résulter non seulement de l'organisation anatomiquefonctionnelle unique des noyaux du tronc cérébral trigéminé, mais aussi de l'interaction entre les mécanismes centraux ascendant et descendant.

Les troubles douloureux orofaciaux constituent une composante majeure et coûteuse des soins de santé et présentent un taux de prévalence élevé avec un impact proportionnellement dévastateur sur la qualité de vie (Département américain de la Santé et des Services Humains, 2000), "... la santé bucco-dentaire signifie beaucoup plus que des dents saines. Cela signifie être exempt de problèmes de douleur orale et faciale chroniques ... ". Les enquêtes communautaires indiquent que de nombreux sujets signalent généralement des douleurs dans la région orofaciale, avec des estimations de plus de 39 millions, soit 22% de la population adulte, uniquement pour les États-Unis (Lipton et al., J Am Dental Assoc (1993), 124 :115-121). D'autres enquêtes sur la population menées au Royaume-Uni (Macfarlane et al., Pain (2002), 99 : 453-458), en Allemagne (Torsten et al., Pain (2003), 102 : 257-263) ou dans des centres régionaux de soins de la douleur aux États-Unis (Dworkin et al., J Am DentAssoc (1990), 120 : 273-281) rapportent des taux similaires (Pau et al., Oral Health & Preventive Dentistry (2003), 1 : 209-220). En outre, la douleur orofaciale provient de nombreux tissus cibles spécifiques, tels que les méninges, la cornée, la pulpe dentaire, la muqueuse buccale et nasale, l'articulation temporo-mandibulaire et présente donc plusieurs caractéristiques physiologiques particulières par rapport au système nociceptif lié à la moelle épinière (Bereiter et al., The senses, a comprehensive reference (2008), vol. 5 : 435-460 Academic Press San Diego, CA).

La névralgie du trijumeau (NT) est une cause fréquente de céphalée aigüe récidivante, concernant particulièrement les femmes à partir de 50 ans. Elle entraine des douleurs importantes, qui peuvent être très handicapantes et source de détresse psychologique majeure dans les cas sévères. Son incidence est d'environ une vingtaine de nouveaux cas annuels pour 100 000 habitants. Elle est deux fois plus fréquente chez la femme et sa fréquence augmente avec l'âge.

Il existe 2 types de névralgies du trijumeau. La névralgie essentielle où les douleurs sont unilatérales, intéressant une partie du visage correspondant à l'un des territoires sensitifs du trijumeau. Elles sont fulgurantes, très intenses, à type de brûlures ou de décharges électriques, durant typiquement de quelques secondes à quelques minutes. Les névralgies symptomatiques du trijumeau ne sont, quant à elles, généralement pas paroxystiques mais avec un fond douloureux. Elles intéressent plusieurs territoires du nerf trijumeau.

Autres douleurs associées au nerf trijumeau, les migraines représentent une des maladies humaines les plus fréquentes, concernant 10 à 15% de la population adulte avec une nette prédominance chez la femme. La migraine est un trouble neurologique associé à des manifestations épisodiques, des attaques, typiquement caractérisées par des maux de tête sévères d'une durée de 4 à 72 heures, accompagnés de dysfonctionnements du système nerveux autonome et divers troubles neurologiques comme l'allodynie cutanée (Goadsby et al., Physiol. Rev. (2017), 97 : 553-622). Lorsque ces troubles sont ponctuels, aléatoires, sans périodicité et que leur nombre n'augmente pas avec le temps, on parle de migraines aiguës. Dans certains cas, la fréquence des attaques augmente, entraînant une chronicisation de la migraine, une forme très invalidante définie par des maux de tête pendant 15 jours ou plus par mois. On parle alors de migraine chronique.

Les crises migraineuses entraînent souvent une difficulté, voire une incapacité à vivre normalement. Les migraineux s'isolent souvent dans un endroit calme et sombre. Le fardeau financier est également énorme pour la société. L'utilisation annuelle de soins de santé peut dépasser un milliard de dollars pour les patients migraineux, tandis que la perte de productivité des employeurs peut atteindre des milliards de dollars (Hu XH et al., Arch Intern Med (1999), 159 (8) : 813-818). Certaines comorbidités sont présentes chez les patients migraineux avec une prévalence plus élevée, comme les accidents vasculaires cérébraux, l'angine de poitrine, l'épilepsie, et certains troubles psychiatriques comme la dépression et l'anxiété.

Des études cliniques ont identifié les facteurs de risque de chronicisation de la migraine. L'un est la fréquence des attaques de maux de tête (Scher IA et al., Pain (2003), 106 : 81-89), un autre est l'allodynie cutanée. Les deux tiers des migraineux présentent une allodynie, pendant (Ashkenasi A et al., Cephalalgia (2007), 27; 111-117) mais aussi entre les crises de migraine (Schwedt TJ et al., Cephalalgia (2011), 31 : 6-12). Le développement et la sévérité de l'allodynie cutanée sont associées à des crises de migraine fréquentes et sont prédictifs de chronicisation de la migraine (Bigal ME et al., Curr Opin Neurol (2009), 22 : 269-276).

Le traitement des migraines doit être instauré dès le début de la crise, le plus tôt possible. Les antalgiques de grade 1 et les AINS (anti-inflammatoires non stéroïdiens) sont suffisants pour les formes modérées. Pour les patients dont les crises sont plus intenses, le traitement nécessite l'usage de triptans, comme le sumatriptan, par voie orale ou injectable, pour lutter directement contre les maux de tête.

Dans le cas des migraines chroniques, des agents prophylactiques entraînant une réduction de la fréquence et de la gravité des attaques sont plutôt prescrits. Le propranolol, un béta-bloquant, est depuis longtemps l'un des médicaments préventifs les plus utilisés (Loder E et al., Headache (2012), 52 : 930-945), ce traitement réduisant la fréquence des maux de tête chez près de 50% des patients (Stovner LJ et al., Cephalalgia (2014), 34 : 523-532). En revanche, son mécanisme et son site d'action restent peu connus.

Alors que des millions d'Américains souffrent de migraines, environ 3% à 13% d'entre eux seulement suivent un traitement préventif, alors qu'environ 38% auraient besoin d'un agent préventif (Lipton RB et al., Neurology (2007), 68(5) 343-349). Les interventions pharmacologiques dans le traitement des migraines comprennent un traitement aigu, dans le but de mettre fin aux attaques, et un traitement préventif quotidien. Le US Headache Consortium de 2000 a défini les objectifs suivants en matière de traitement préventif : (1) réduire la fréquence des attaques de 50% et en diminuer l'intensité et la durée ; (2) améliorer la réactivité au traitement aigu ; (3) améliorer la fonction et réduire l'invalidité ; et (4) prévenir la survenue de céphalées dues à une utilisation excessive de médicaments analgésiques et prévenir la survenue de céphalées chroniques. Les bêta-bloquants comme le propranolol sont la classe la plus largement utilisée de médicaments prophylactiques de la migraine et sont efficaces dans 50 % des cas mais d'autres comme les antidépresseurs tricycliques, les antagonistes des canaux calciques, les antagonistes CGRP, les antiépileptiques, les anti-inflammatoires non stéroïdiens peuvent également être utilisés (Silberstein SD, Continuum (2015), 21(4) : 973-989).

Des affections comme la sclérose en plaques peuvent causer des névralgies secondaires du trijumeau.

Le traitement de la névralgie du trijumeau essentielle est d'abord médical, parfois chirurgical. Le traitement médical fait appel, en première intention, à la carbamazépine dont les doses sont augmentées progressivement jusqu'à la dose minimale efficace. D'autres molécules sont utilisées : l'oxcarbazépine, le baclofène, le clonazépam, la lamotrigine ou encore la gabapentine ou la prégabaline.

Divers médicaments et procédures chirurgicales ont été utilisés pour le traitement de la névralgie du trijumeau (NT). Malgré les nombreuses approches disponibles, les résultats sont loin d'être satisfaisants. En outre, un certain nombre de patients deviennent résistants aux médicaments, ce qui nécessite une intervention chirurgicale pour traiter la névralgie. Néanmoins, la douleur récidive fréquemment après une ou plusieurs opérations chirurgicales (Montano et al., Ther Clin Risk Manag (2015), 11 : 289-299).

La douleur liée à la sclérose en plaques peut être d'origine nociceptive, neuropathique ou être due à un mélange de celles-ci. Des conditions spécifiques incluent la névralgie du trijumeau et le phénomène de Lhermitte. Celles-ci sont associées à des douleurs extrêmes, des spasmes toniques douloureux, des douleurs associées au nerf optique, des douleurs musculaires et des migraines (Truini et al., J Neurol (2013), 260 : 351-367).

L'encéphalite auto-immune expérimentale (EAE) est le modèle expérimental le plus utilisé de sclérose en plaques. Il partage de nombreuses caractéristiques pathologiques avec les cas humains comme la neuro-inflammation, la démyélinisation et les dommages aux neurones (Duffy et al., Mult Scler Int (2014) 285245. doi : 10.1155/2014/285245). Bien que cela ne soit pas totalement clair, il semble que la douleur générée dans ce modèle soit le résultat d'une inflammation, d'une activation gliale et d'une démyélinisation (Olechowski et al., Pain (2009), 141 : 156-164) ainsi qu'un processus lié au nerf trijumeau (Thorburn et al., Pain (2016), 157(3) : 627-642).

### Effet analgésique des enképhalines

La perception, la transmission et la régulation des influx nociceptifs sont sous la dépendance de plusieurs neurotransmetteurs, et en particulier les enképhalines (Metenképhaline et Leu-enképhaline). Celles-ci sont des pentapeptides, opioïdes endogènes, initialement trouvés dans le cerveau de mammifères (Hugues et al., Nature (1975), 258, 577-579). Elles se lient principalement à deux classes de récepteurs, les récepteurs opioïdes µ et δ (Waterfield et al., Eur J Pharmacol (1979), 58 : 11-18) dont les fonctions et les localisations sont différentes (Waksman et al., Proc Natl Acad Sci USA (1986), 83: 1523-1527).

Les propriétés antinociceptives des enképhalines ont été démontrées après administration par voie intracérébroventriculaire d'enképhalines exogènes (Belluzi et al., Nature (1976), 260 : 625-626). Cependant, cette réponse est très fugace à cause d'une métabolisation très rapide de ces peptides par des enzymes. Des analogues d'enképhalines synthétiques, modifiés pour les rendre résistants à la dégradation enzymatique ont montré des propriétés antinociceptives égales à celles de la morphine, mais ont également présenté les mêmes effets secondaires indésirables que celle-ci.

D'autre part, on sait que les enképhalines (Tyr-Gly-Gly-Phe-Met et Tyr-Gly-Gly-Phe-Leu) sont physiologiquement inactivées par deux métallopeptidases à zinc, la néprilysine (EC 3.4.24.11, NEP) qui clive la liaison Gly³-Phe⁴ (Malfroy et al., Nature (1978), 276 : 523-526) et l'aminopeptidase N (EC 3.4.11.2, APN) qui coupe la liaison Tyr¹-Gly² de ces peptides (Waksman et al., Eur J Pharmacol (1985), 117 : 233-243).

L'inhibition de ces deux activités enzymatiques, en protégeant complètement les enképhalines (Bourgoin et al., J Pharm Exp Ther (1986), 238 (1), 360-366), révèlent les activités pharmacologiques et en particulier analgésiques et antidépressives (Roques, Trends PharmacolSci (2000), 21, 475-483 ; Jutkiewicz, CNS Drug Rev (2007), 13, 192-205) des opioïdes endogènes, les enképhalines.

### Interactions entre le système enképhalinergique et le nerf trijumeau

Les récepteurs opioïdes sont présents dans le noyau caudal du trijumeau du chat et du rat (Wang, J Neurophysiol (2000), 83 : 3570-3574).

Les récepteurs delta sont impliqués dans le mécanisme de migraine (Charles & Pradhan, Curr Opin (2016), 29 : 314-319). En effet, des agonistes delta (SNC80 par exemple) inhibent l'hyperalgésie liée à des cas de migraines chez la souris (Pradhan et al., Br J Pharmacol (2014), 171 : 2375-2384).

Il a été montré dans le cas de neuropathies liées au nerf trijumeau qu'il y avait une activation continue de la neurotransmission des opioïdes endogènes passant principalement par une activation des récepteurs mu (Da Silva et al., Curr Pain Headache Rep (2014), 18: 429. doi : 10.1007/s11916-014-0429-0). De la même manière, il a été observé une augmentation du niveau de Met-ENK chez des patients migraineux (Mosnaim et al., Headache (1986), 26 : 278-281).

Dans le cas de la migraine, les récepteurs mu modulent les signaux nociceptifs (Storer et al., Br J Pharmacol (2003), 138 : 317-324). En effet, il a été montré qu'une mutation du gène des récepteurs mu augmentait la sensibilité aux crises de migraine (Menon et al., Pain (2012), 13 : 513-519). Une action périphérique d'agoniste mu a également été démontré (Baillie et al., Neuropharmacol (2015), 93 : 267-273).

Les opioïdes (morphine, codéine et péthidine) sont efficaces dans la migraine (Siberstein, Cephalalgia (2000), 20 : 854-864). En effet, il a été démontré que des agonistes opioïdes inhibaient la vasodilatation méningée neurogénique (Williamson et al., Br J Pharmacol (2001), 133 : 807-814) comme le font les triptans.

Le transfert à l'aide de l'herpès simplex virus type-1 (HSV-1) permet la surexpression de pro-enképhalines dans les neurones primaires sensoriels au niveau lombaire. Une telle administration a permis de montrer un effet anti-hyperalgésique dans un modèle de douleur chronique chez le rat ou dans un modèle de douleur neuropathique consécutive à une ligature du nerf spinal (Hao et al., Pain (2003), 102 : 135-142). Au contraire de la morphine, ce genre de traitement n'a pas montré de tolérance après 4 semaines de traitement ou après 8 semaines de traitement (Hao et al., Pain (2003), 102 : 135-142). Par contre, de la résistance a été observée lors de traitement à la morphine (Meunier et al., Mol Therap (2005), 11 : 608-616).

Il existe donc un besoin pour une thérapie préventive et des traitements améliorés des douleurs relatives au nerf trijumeau, et notamment les migraines ou les névralgies du trijumeau, qui permettraient d'éviter les effets secondaires des traitements connus, en particulier les phénomènes d'addiction et d'accoutumance. Dans le cas de la migraine chronique par exemple, avec un médicament préventif, la fréquence des migraines peut être réduite et la réponse au traitement aigu améliorée, ce qui permet une réduction des dépenses en soins de santé, du nombre de journées de travail perdues et une amélioration de la qualité de vie des patients.

### RESUME DE L'INVENTION

Des inhibiteurs mixtes de la dégradation enzymatique des enképhalines endogènes ont permis de révéler complétement les activités pharmacologiques et en particulier analgésiques et antidépressives des enképhalines. Différentes séries d'inhibiteurs mixtes ont été décrits dans différents brevets (WO 98/18803 ; WO 2009/138436 ; WO 2010/010106 ; WO 2014/064166).

Une bonne efficacité antinociceptive a été démontrée sur de nombreux modèles de douleurs aiguës ou neuropathiques (Poras et al., J Med Chem (2014), 57 : 5748-5763 ; Bonnard et al., Pharma Res Per (2015), 3(2), e00116, doi: 10.1002/prp2.116; Poras et al., Eur J Med Chem (2015), 102: 58-67). Toutefois, l'utilisation de ces composés comme analgésiques agissant sur des douleurs spécifiquement relatives au nerf trijumeau, comme la migraine ou la névralgie du trijumeau, n'a jamais été décrite ou suggérée.

Or, de manière surprenante, les inventeurs ont démontré que des dérivés de type aminoacides contenant un groupement disulfanyle - décrits en tant qu'inhibiteurs mixtes de NEP et d'APN et présentant des activités analgésiques dans des modèles de douleurs centrales ou périphériques après une administration intraveineuse ou orale - sont efficaces pour la prévention et le traitement des douleurs relatives au nerf trijumeau, en particulier pour le traitement des migraines, qu'elles soient chroniques ou aiguës.

Selon un autre mode de réalisation, les dérivés de types aminoacides contenant un groupement disulfanyle selon l'invention sont efficaces dans le traitement et la prévention des crises migraineuses.

Contrairement à la méthode impliquant le transfert à l'aide de l'herpès simplex virus type-1 (HSV-1) citée précédemment, une surexpression d'enképhaline au sein du système nerveux n'est pas recherchée via l'utilisation de ces inhibiteurs mixtes de NEP et d'APN.

L'invention a ainsi pour objet un sel pharmaceutiquement acceptable d'un composé de formule (I) pour prévenir ou traiter des douleurs relatives au nerf trijumeau, en particulier les migraines ou les névralgies du trijumeau :

H₂N-CH(R₁)-CH₂-S-S-CH₂-CH(R₂)-CONH-CH(R₃)-COOR₄ (I)

dans laquelle :
∘ R₁ représente :
   - une chaîne hydrocarbonée, saturée ou insaturée, linéaire ou ramifiée, comportant de 1 à 6 atomes de carbones, éventuellement substituée par :
      * un radical OR, SR ou S(O)R, dans chacun de ces radicaux R représente un hydrogène, une chaîne hydrocarbonée linéaire ou ramifiée de 1 à 4 atomes de carbone, un radical phényle ou benzyle,
      * un radical phényle ou benzyle,
   - un radical phényle ou benzyle éventuellement substitué par :
      * 1 à 5 halogènes, notamment le fluor,
      * un radical OR, SR ou S(O)R, dans chacun de ces radicaux R ayant la même signification que précédemment,
   - un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde ;
o R₂ représente :
   - un radical phényle ou benzyle, éventuellement substitué par :
      * 1 à 5 atomes d'halogènes notamment le fluor,
      * un radical OR ou SR, dans chacun de ces radicaux R ayant la même signification que précédemment,
      * un groupement amino éventuellement mono- ou di-substitué par un groupement aliphatique, cyclique ou linéaire, de 1 à 6 atomes de carbones,
      * un cycle aromatique à 5 ou 6 atomes,
   - un hétérocycle aromatique à 5 ou 6 atomes, l'hétéroatome étant un oxygène, un azote ou un soufre,
   - un groupe méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme de N-oxyde ou de S-oxyde ;
∘ R₃ représente :
   - un hydrogène,
   - un groupement OH ou OR, R ayant la même signification que précédemment,
   - une chaîne hydrocarbonée saturée (alkyle), linéaire ou ramifiée, comportant de 1 à 6 atomes de carbone, éventuellement substitué par un radical OR ou SR, dans chacun de ces radicaux R a la même signification que précédemment,
   - un radical phényle ou benzyle, éventuellement substitué par :
      * 1 à 5 halogènes notamment le fluor,
      * un radical OR ou SR, R ayant la même définition que précédemment. et
∘ OR₄ représente
   - un radical glycolate OCH₂COOR' ou lactate OCH(CH₃)COOR', dans chacun de ces radicaux R' représente :
      - une chaîne hydrocarbonée saturée (alkyle) de 1 à 6 atomes de carbone, linéaire ou ramifiée éventuellement substituée par un groupement alkoxy en C₁-C₃, de préférence un groupe alkyle en C₁-C₄ éventuellement substitué par un groupe méthoxy,
      - un groupement cycloalkyle en C₅-C₈, de préférence un groupe cycloalkyle en C₅-C₆,
      - un groupement phényle, benzyle, hétéroaryle, alkylhétéroaryle ;
   - un groupement OCH(R")O(CO)OR' ou OCH(R")O(CO)R', dans chacun de ces radicaux R' a la même signification que précédemment et R" représente :
      - un atome d'hydrogène,
      - une chaîne alkyle en C₁-C₆ linéaire ou ramifiée éventuellement substituée par un groupement alkoxy de C₁-C₃, de préférence un groupe alkyle en C₁-C₄ éventuellement substitué par un groupe méthoxy,
      - un groupe cycloalkyle de C₅-C₈, de préférence un groupe cycloalkyle en C₅-C₆,
      - un groupement phényle, benzyle, hétéroaryle, alkylhétéroaryle ;
   - un radical triglycéride OCH(CH₂OCOR')₂ ou OCH₂-CH(OCOR')-CH₂OCOR', dans chacun de ces radicaux R' ayant la même signification que précédemment ;
   - un radical glycoside tel que D-glucose, β-D-glucopyranose, α- ou β-galactopyranose ;
   - un radical sulfonate OCH₂CH₂(SO₂)CH_{3;}
   - un radical OCH(CH₂OH)₂.

La présente invention a également trait à une composition, notamment pour son utilisation pour prévenir ou soulager (traiter) les douleurs relatives au nerf trijumeau, ladite composition comprenant à titre de principe actif au moins un sel pharmaceutiquement acceptable d'un composé de formule (I) de l'invention, et au moins un excipient pharmaceutiquement acceptable.

La présente invention a également trait à une composition, notamment pour son utilisation pour prévenir les crises de migraine, ladite composition comprenant à titre de principe actif au moins un sel pharmaceutiquement acceptable d'un composé de formule (I) de l'invention, et au moins un excipient pharmaceutiquement acceptable.

La présente invention a également trait à l'utilisation d'un sel pharmaceutiquement acceptable d'un composé de formule (I), ou une composition le comprenant, pour la fabrication d'un médicament pour prévenir ou soulager (traiter) les douleurs relatives au nerf trijumeau, en particulier les migraines ou les névralgies du trijumeau.

La présente invention a également trait à l'utilisation d'un sel pharmaceutiquement acceptable d'un composé de formule (I), ou une composition le comprenant, pour la fabrication d'un médicament pour prévenir les crises de migraine.

La présente invention a également trait à un sel pharmaceutiquement acceptable d'un composé de formule (I) pour son utilisation dans une méthode de prévention ou de traitement des douleurs relatives au nerf trijumeau, en particulier les migraines ou les névralgies du trijumeau, comprenant l'administration d'une dose efficace de sel pharmaceutiquement acceptable d'un composé de formule (I), ou d'une composition le comprenant, à un patient en ayant besoin.

La présente invention a également trait à un sel pharmaceutiquement acceptable d'un composé de formule (I) pour son utilisation dans une méthode de prévention des crises de migraine, comprenant l'administration d'une dose efficace de sel pharmaceutiquement acceptable d'un composé de formule (I), ou d'une composition le comprenant, à un patient en ayant besoin.

La présente invention a également trait à une composition comprenant une quantité comprise entre 50 mg et 800 mg, d'un sel d'un composé de formule (I) ou d'un sel de formule (II) selon les revendications.

Dans la présente invention, le patient (souffrant de douleurs relatives au nerf trijumeau, en particulier de crises migraineuses) est typiquement un animal, de préférence un mammifère, avantageusement il s'agit d'un homme.

### DESCRIPTION DETAILLEE

Dans le cadre de la présente invention, on entend désigner par « douleur relative au nerf trijumeau » une douleur relative à la partie sensitive du trijumeau. Une « douleur relative à la partie sensitive du trijumeau » recouvre selon la présente invention différents types de douleur ayant pour origine la partie sensitive du nerf trijumeau. Il s'agit en particulier de douleurs crânio-faciales, et en particulier de douleurs oro-faciales. Il peut s'agir de douleurs localisées dans la cornée, les méninges ou la pulpe dentaire, les dermatomes du visage, les issus muqueux, les tissus vasculaires, ou les tissus musculaires. Il peut également s'agir de douleurs localisées dans la cornée, la conjonctive, le nez, la muqueuse intranasale, les paupières, la peau supra-orbitaire, le front, le cuir chevelu, les lèvres, la mâchoire, la langue, les gencives, la muqueuse buccale.

En particulier, il s'agit de la migraine ou de la névralgie du trijumeau, notamment la névralgie essentielle et la névralgie symptomatique du trijumeau. Il peut également s'agir de céphalées, notamment de céphalées aiguës, en particulier récidivantes, ou de leurs symptômes telle l'allodynie cutanée céphalique. Il peut également s'agir de douleur périphérique trigéminale associée à la sclérose en plaque.

Dans le cadre de la présente invention, le terme « migraine » désigne à la fois la migraine aiguë, qui définit un mal de tête ponctuel d'une durée de 4 à 72h survenant moins de 15 jours par mois, et la migraine chronique, qui se caractérise par une chronicité des céphalées, avec des maux de têtes qui apparaissent de manière récurrente soit sans raison apparente, soit suite à l'exposition du patient à un facteur déclencheur (comme la lumière vive, les menstruations etc.) quinze jours ou plus par mois (Headache Classification Committee of the International Headache Society (IHS). The International Classification of Headache Disorders, 3rd edition, Cephalalgia (2013), 33 : 629-808).

Dans le cadre de la présente invention, les termes « crises migraineuses », « crises de migraines » ou « migraine chronique » sont interchangeables.

Par traitement, on entend au sens de la présente invention, l'atténuement ou la disparition des douleurs. Dans ce cas, le composé ou la composition selon l'invention est administré après l'apparition des douleurs.

Par prévention, on entend au sens de la présente invention que la douleur n'est pas encore apparue et que le composé ou la composition selon l'invention est administré avant l'apparition des douleurs, empêchant ainsi ladite apparition des douleurs ou atténuant les douleurs à venir.

Par « dose thérapeutique », on entend, au sens de la présente invention, la quantité de principe actif (par exemple de sel de composé de formule (I)) quotidienne administrée au patient en ayant besoin.

Par « de manière répétée », on entend que la dose thérapeutique ou les compositions de l'invention le cas échéant sont administrées par prises décalées dans le temps pendant une période de temps allant de plusieurs jours à plusieurs mois, le plus souvent avec des intervalles de temps réguliers entre les prises.

Par groupe « amino », on entend, au sens de la présente invention, un groupe de formule -NR*R**, où R* et R** représentent, indépendamment l'un de l'autre, un atome d'hydrogène ou un groupe hydrocarboné saturé ou insaturé, linéaire, ramifié ou cyclique, comportant de 1 à 6, de préférence de 1 à 4, atomes de carbone, ou R* et R** forment ensemble, avec l'atome d'azote qui les porte, un hétérocycle à 5 ou 6 chaînons, saturé ou non, et ne comportant pas d'autre hétéroatome que l'azote qui porte les deux radicaux R* et R**. En particulier, le groupe amino peut être un groupe -NH₂, -NHMe, - NHEt, -NHPr, NHiPr, -NHBu, -NHiBu, -NHtBu, pipéridinyle ou pyrrolidinyle. De préférence, un groupe amino est un groupe de formule -NR*R**, où R* et R** représentent, indépendamment l'un de l'autre, un atome d'hydrogène ou un groupe alkyle en C₁-C₆ (de préférence en C₁-C₄) linéaire ou ramifié.

Dans le cadre de la présente invention, l'expression « chaîne hydrocarbonée » désigne des alcanes, des alcènes ou des alcynes. En particulier, l'expression « chaîne hydrocarbonée saturée » désigne des radicaux alkyles comportant de 1 à 6 atomes de carbone (C1-C6) ou de 1 à 4 atomes de carbone (C₁-C₄), linéaires ou ramifiés. Comme exemple de radicaux alkyles comportant de 1 à 4 atomes de carbones, on peut citer les radicaux méthyl, éthyl, propyl, butyl, isopropyl, 1-méthyl-éthyl, 1-méthyl-propyl, 2-méthyl-propyl. Comme exemple de radicaux alkyles comportant de 1 à 6 atomes de carbones, on peut en outre citer les radicaux pentyl, hexyl 1-méthyl-butyl, 1-méthyl-pentyl 2-méthyl-butyl 2-méthyl-pentyl, 3-méthyl-butyl, 3-méthyl-pentyl, 4-méthyl-pentyl ou 1-éthyl-propyl, 1-éthyl-butyl, 2-éthyl-butyl. L'expression « chaîne hydrocarbonée insaturée » désigne des radicaux alcényle (au moins une double liaison), par exemple vinyle, allyle ou analogue, ou alcynyle (au moins une triple liaison) comportant de 2 à 6 atomes de carbone, ou de 2 à 4 atomes de carbone, linéaires ou ramifiés.

Par « cycloalkyle », on entend, au sens de la présente invention, un cycle hydrocarboné saturé comportant de 5 à 8 atomes de carbone, en particulier le groupe cyclohexyle, cyclopentyle ou cycloheptyle.

Le terme « halogène » utilisé ici désigne un chlore, un brome, un iode et un fluor. Avantageusement, il s'agit d'un atome de fluor, de brome ou de chlore. Encore avantageusement, il s'agit d'un atome de fluor ou de brome, et de préférence de fluor.

Par groupement ou radical « aromatique », on entend, au sens de la présente invention, un groupement aromatique, comportant de préférence de 5 à 10 atomes de carbone, sauf mention contraire, et comprenant un ou plusieurs cycles accolés, comme par exemple un groupement phényle ou naphtyle. Avantageusement, il s'agit du phényle.

Par groupement ou radical « hétéroaromatique », on entend, au sens de la présente invention, tout groupe aromatique tel que défini ci-dessus dans lequel un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydé sous forme de S-oxyde ou de N-oxyde. Des exemples de groupes hétéroaromatique sont les groupes furyle, thiényle, pyrrolyle, pyridinyle, pyrimidyle, pyrazolyle, imidazolyle, tétrazolyle ou encore indyle. De préférence, il s'agit d'un groupe thiényle.

Par « cycle hétéroaromatique à 5 ou 6 atomes », on entend, au sens de la présente invention, un groupe hétéroaromatique tel que défini ci-dessus ne comportant qu'un seul cycle à 5 ou 6 atomes. Il s'agit notamment d'un groupe thiényle, pyrrolyle, pyridinyle, pyrimidyle, pyrazolyle, imidazolyle ou encore tétrazolyle.

Par « hétérocycle », on entend, au sens de la présente invention, un cycle hydrocarboné, avantageusement à 5 ou 6 atomes, dont un ou plusieurs atome(s) de carbone a(ont) été remplacé(s) par un ou plusieurs hétéroatome(s), avantageusement 1 à 4 et, encore plus avantageusement 1 à 2, tels que par exemple des atomes de soufre, azote ou oxygène, les atomes de soufre et d'azote pouvant être éventuellement oxydés sous forme de N-oxyde et de S-oxyde. Sauf mention contraire, ce cycle pourra être saturé ou aromatique. Comme exemple de noyaux hétérocycliques à 5 ou 6 atomes, aromatiques ou saturés, possédant comme hétéroatome un atome d'azote ou de soufre, on peut citer, mais sans limitation, les radicaux suivants : thiényl, pyrrolyl, imidazolyl, pyrazolyl, isothiazolyl, pyridyl, pyrazinyl, pyrimidinyl, pyridazinyl, pyrrolidinyl, pyrrolinyl, imidazolidinyl, pyrazolidinyl, pyrazolinyl, piperidyl, piperazinyl, thiadiazolyle, les atomes d'azote et de soufre étant éventuellement oxydés sous forme de N-oxyde ou de S-oxyde. Comme exemple de noyaux hétérocycliques à 5 ou 6 atomes, aromatiques ou saturés, possédant comme hétéroatome un atome d'oxygène, on peut citer, mais sans limitation, les radicaux suivants : furyl, pyranyl, isoxazolyl, morpholinyl, furazanyl, oxazolyl, oxazolidinyl, oxazolinyl.

Les composés de formule (I) ont potentiellement de 2 à 9 centres d'asymétrie. Les radicaux R₁, R₂ et R₃ seront typiquement introduits de façon à obtenir des enchaînements optiquement purs correspondant à une stéréochimie reconnue par les activités enzymatiques. Les radicaux R₄ pourront éventuellement contenir un centre d'asymétrie non résolu.

Les composés de l'invention sont sous forme de sels pharmaceutiquement acceptables, ou d'un hydrate de ceux-ci. Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les sels d'addition d'acide pharmaceutiquement acceptable formés avec des acides, et
(2) les hydrates et les solvates de ceux-ci.

Typiquement, les composés de formule (I) sont sous forme de sels d'addition obtenus avec des acides organiques ou minéraux pharmacologiquement acceptables tels que les phosphates, le chlorhydrate, l'acétate, le méthanesulfonate, le borate, le lactate, le fumarate, le succinate, l'hémisuccinate, le citrate, le tartrate, l'hémitartrate, le maléate, l'ascorbate, l'hemifumarate, l'hexanoate, l'heptanoate, l'hippurate, l'hydrocinnamate, le phénylglyoxylate et le nicotinate.

Le solvate est de préférence un alcoolate, tel qu'un éthanolate.

De préférence, les composés de l'invention sont sous forme de sels de fumarate ou d'un hydrate de ceux-ci.

Le radical R₁ représente avantageusement un radical alkyle ayant de 1 à 6 ou de 1 à 4 atomes de carbone, éventuellement substitué par un radical OR, SR ou S(O)R, dans chacun de ces radicaux R la même signification que précédemment, et représente avantageusement un hydrogène, une chaîne hydrocarbonée linéaire ou ramifiée de 1 à 4 carbones, un radical phényle ou benzyle. R₁ représente encore plus avantageusement un radical alkyle ayant de 1 à 4 atomes de carbone substitué par un radical SR, R ayant la même signification que précédemment, en particulier R représente une chaîne hydrocarbonée saturée linéaire ou ramifiée de 1 à 4 atomes de carbone.

Le radical R₂ représente avantageusement :
- un groupe phényle ou benzyle, éventuellement substitué par :
   *1 à 5 atomes d'halogènes notamment le fluor, un hydroxyle ou un thiol, un éther OR ou un thio-ether SR, R ayant la même signification que ci-dessus,
   *un cycle aromatique ou hétérocyclique aromatique à 5 ou 6 atomes, l'hétéroatome étant un oxygène, un azote ou un soufre,
- un groupe méthylène substitué par un hétérocycle à 5 ou 6 atomes, aromatique ou saturé, l'hétéroatome étant un oxygène, un azote ou un soufre, les atomes d'azote et de soufre étant éventuellement oxydés sous forme de N-oxyde ou de S-oxyde.

En particulier, le radical R₂ représente un radical benzyle ou phényle, ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde. En particulier, le radical R₂ représente un radical benzyle ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde, encore plus avantageusement un radical benzyle ou un radical méthylène substitué par un radical thiophényle (thiényle).

Avantageusement, le radical R₃ représente :
- un hydrogène,
- un groupement OH ou OR, R ayant la même signification que ci-dessus,
- un alkyle en C₁-C₆ linéaire ou ramifié, éventuellement substitué par un groupe OH, OR, SH ou SR, R ayant la même signification que ci-dessus,
- un groupe phényle ou benzyle substitué ou non par 1 à 5 halogènes notamment le fluor, par un groupe OR ou SR, R ayant la même définition que ci-dessus.

En particulier, le radical R₃ représente un atome d'hydrogène ou un radical alkyle ayant de 1 à 6 atomes de carbone, encore plus avantageusement 1 à 4 atomes de carbone, éventuellement substitué par un radical OR ou SR, dans chacun de ces radicaux R ayant la même signification que précédemment. Le radical R₃ représente encore plus avantageusement un atome d'hydrogène ou un radical alkyle ayant de 1 à 6 atomes de carbone, encore plus avantageusement 1 à 4 atomes de carbone, substitué par un radical OH ou SH.

Le radical OR₄ représente avantageusement :
- un radical glycolate OCH₂COOR', R' ayant la même signification que précédemment (en particulier R' représente un groupe alkyle en C1-C4 éventuellement substitué par un groupe méthoxy ou un groupe cycloalkyle en C₅-C₆),
- un radical OCH(R")O(CO)OR' ou OCH(R")O(CO)R', R' et R" ayant la même signification que précédemment (en particulier R' et/ou R" représente un groupe alkyle en C₁-C₄ éventuellement substitué par un groupe méthoxy ou un groupe cycloalkyle en C₅-C₆ ou R" représente un atome d'hydrogène),
- un radical triglycéride OCH(CH₂OCOR')₂ ou OCH₂-CH(OCOR')-CH₂OCOR', dans chacun de ces radicaux R' ayant la même signification que précédemment
- un radical glycoside tel que D-glucose,
- un radical sulfonate OCH₂CH₂(SO₂)CH₃,
- un radical OCH(CH₂OH)₂.

Alternativement, le radical OR₄ peut représenter :
- un radical glycolate OCH₂COOR' ou lactate OCH(CH₃)COOR',
- un groupement OCH₂OCOR' ou OCH(CH₃)OCOOR', ou
- un radical triglycéride OCH(CH₂OCOR')₂ ou OCH₂-CH(OCOR')-CH₂OCOR',

R' représentant un groupe alkyle en C₁-C₄ linéaire ou ramifié.

En particulier, le radical OR₄ représente un groupement OCH(R")O(CO)OR' ou OCH(R")O(CO)R', le radical R' représentant une chaîne alkyle en C1-C4 (en particulier le radical éthyle) et le radical R" représentant un radical méthyle, CH(CH₃)₂, cyclohexyle ou phényle.

Selon un mode de réalisation particulier, le sel de l'invention est un sel de la formule (II) :

A-, H₃N⁺-CH(R₁)-CH₂-S-S-CH₂-CH(R₂)-CONH-CH(R₃)-COOR₄ (II)

dans laquelle :
∘ A- représente un anion phosphate, chlorure, acétate, méthanesulfonate, borate, lactate, fumarate, succinate, hémisuccinate, citrate, tartrate, hémitartrate, maléate, ascorbate, hemifumarate, hexanoate, heptanoate, hippurate, hydrocinnamate, phénylglyoxylate ou nicotinate ;
∘ R₁ représente une chaîne hydrocarbonée saturée ou insaturée, linéaire ou ramifiée comportant de 1 à 6 atomes de carbones, substituée ou non par un groupement OR, SR ou S(O)R dans lequel R représente un hydrogène, une chaîne hydrocarbonée linéaire ou ramifiée de 1 à 4 carbones, un radical phényle ou benzyle ;
∘ R₂ représente :
   - un groupe phényle ou benzyle, éventuellement substitué par :
      *1 à 5 atomes d'halogènes notamment le fluor, un hydroxyle ou un thiol, un éther OR ou un thio-ether SR, R ayant la même signification que ci-dessus,
      *un cycle aromatique ou hétérocyclique aromatique à 5 ou 6 atomes, l'hétéroatome étant un oxygène, un azote ou un soufre,
   - un groupe méthylène substitué par un hétérocycle à 5 ou 6 atomes, aromatique ou saturé, l'hétéroatome étant un oxygène, un azote ou un soufre, les atomes d'azote et de soufre étant éventuellement oxydés sous forme de N-oxyde ou de S-oxyde ;
∘ R₃ représente :
   - un hydrogène,
   - un groupement OH ou OR, R ayant la même signification que ci-dessus,
   - un alkyle en C₁-C₆ linéaire ou ramifié, éventuellement substitué par un groupe OH, OR, SH ou SR, R ayant la même signification que ci-dessus,
   - un groupe phényle ou benzyle substitué ou non par 1 à 5 halogènes notamment le fluor, par un groupe OR ou SR, R ayant la même définition que ci-dessus ;
∘ OR₄ représente :
   - un radical glycolate OCH₂COOR' ou lactate OCH(CH₃)COOR',
   - un groupement OCH₂OCOR' ou OCH(CH₃)OCOOR', ou
   - un radical triglycéride OCH(CH₂OCOR')₂ ou OCH₂-CH(OCOR')-CH₂OCOR', R' représentant un alkyle en C₁-C₄ linéaire ou ramifié.

De préférence, dans la formule (II), A⁻ représente un anion fumarate.

Toutes les combinaisons des modes de réalisation particuliers, avantageux et préférés des substituants R₁, R₂, R₃ et R₄ dans les formules I et II sont envisagées dans la présente invention.

L'invention concerne en particulier les sels d'addition d'acide des composés suivants, et en particulier leurs sels de fumarate :
1-(2-(1-(2,3-diacétoxypropoxycarbonyl)-éthylcarbamoyl)-3-thiophèn-3-ylpropyl disulfanylméthyl)-3-méthylsulfanylpropyl-amine,
1-(2-(1-(2-méthanesulfonyléthoxycarbonyl)-éthylcarbamoyl)-3-thiophèn-3-ylpropyl disulfanylméthyl)-3-méthylsulfanylpropyl- amine,
1-(2-(1-(1-éthoxycarbonyloxyéthoxycarbonyl))-éthylcarbamoyl)-3-thiophèn-3-yl-propyl disulfanylméthyl)-3-méthylsulfanylpropyl- amine,
1-(2-(1-éthoxycarbonylméthyloxycarbonyléthylcarbamoyl)-3-thiophèn-3-yl-propyl disulfanylméthyl)-3-méthylsulfanylpropyl- amine,
1-(2-(1-(1-éthoxycarbonyloxyéthoxycarbonyl)-2-hydroxypropylcarbamoyl)-3-thiophèn-3-ylpropyldisulfanylméthyl)-3-méthylsulfanylpropyl- amine,
1-(2-(1-(2-acétoxy-1-acétoxyméthyléthoxycarbonyl)-éthylcarbamoyl)-3-thiophèn-3-yl propyldisulfanylméthyl)-3-méthylsulfanylpropyl- amine,
1-(2-(1-(2-hydroxy-1-hydroxyméthyléthoxycarbonyl)-éthylcarbamoyl)-3-thiophèn-3-yl propyldisulfanylméthyl)-3-méthylsulfanylpropyl- amine,
1-(2-(1-(3,4,5,6-tétrahydroxytétrahydropyran-2-ylméthoxycarbonyl)-éthylcarbamoyl)-3-thiophèn-3-yl-propyldisulfanylméthyl)-3-méthylsulfanylpropyl- amine,
1-(2-(1-(1-éthoxycarbonyloxy-éthoxycarbonyl)-2-hydroxypropylcarbamoyl)-3-phényl propyldisulfanylméthyl)-3-méthylsulfanylpropyl- amine,
1-(2-(1-(2-acétoxy-1-acétoxyméthyl-éthoxycarbonyl)-2-hydroxypropylcarbamoyl)-3-phénylpropyldisulfanylméthyl)-3-méthylsulfanylpropyl-amine,
1-(2-((1-éthoxycarbonyloxy-éthoxycarbonylméthyl)-carbamoyl)-3-phényl-propyl disulfanylméthyl)-3-méthylsulfanylpropyl- amine,
3-(2-amino-4-méthylsulfanyl-butyldisulfanyl)-2-benzyl-N-(5-éthyl-(1,3,4)-thiadiazol-2-yl)-propionamide,
1-(2-((1-éthoxycarbonyloxy-2-méthyl-propoxycarbonylméthyl)-carbamoyl)-3-phényl-propyldisulfanylméthyl)-3-méthylsulfanyl-propyl-amine,
1-(2-((cyclohexyl-éthoxycarbonyloxy-méthoxycarbonylméthyl)-carbamoyl)-3-phényl-propyldisulfanylméthyl)-3-méthylsulfanyl-propyl-amine,
1-(2-((éthoxycarbonyloxy-phényl-méthoxycarbonylméthyl)-carbamoyl)-3-phényl-propyldisulfanylméthyl)-3-méthylsulfanyl-propyl-amine,
3-méthylsulfanyl-1-(3-phényl-2-((1-propionyloxy-éthoxycarbonylméthyl)-carbamoyl)-propyldisulfanylméthyl)-propyl-amine,
1-(2-((2-méthyl-1-propionyloxy-propoxycarbonylméthyl)-carbamoyl)-3-phényl-propyldisulfanylméthyl)-3-méthylsulfanyl-propyl-amine,
1-(2-((cyclohexyl-propionyloxy-méthoxycarbonylméthyl)-carbamoyl)-3-phényl-propyldisulfanylméthyl)-3-méthylsulfanyl-propyl-amine,
3-méthylsulfanyl-1-(3-phényl-2-((phényl-propionyloxy-méthoxycarbonylméthyl)-carbamoyl)-propyl disulfanylméthyl)-propyl-amine.

De manière préférée, le composé de l'invention est le (5S, 10S)-10-benzyl-16-methyl-11,14,18-trioxo-15,17,19-trioxa-2,7,8-trithia-12-azahenicosan-5-aminium fumarate (ou (E)-3-carboxyacrylate) (ci-après composé 1):

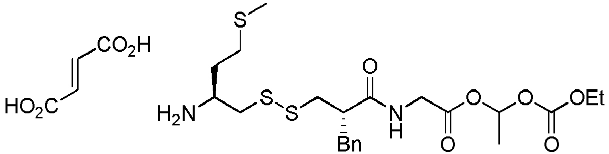

Les sels des composés de formule (I) peuvent être synthétisés, par exemple, par des méthodes décrites dans WO 2007/048787. Plus particulièrement, le composé 1 peut être synthétisé comme décrit dans WO 2017/064250.

Les composés de formule (I) sont formulés en accord avec des méthodes décrites par l'homme de l'art, en particulier pour la voie d'administration désirée.

L'invention a aussi pour objet les compositions pharmaceutiques contenant à titre de principe actif au moins un sel pharmaceutiquement acceptable d'un composé de formule générale (I) ou hydrates de ces sels en combinaison avec un ou plusieurs supports inertes ou autres véhicules pharmaceutiquement acceptables, pour leur utilisation pour prévenir ou soulager des douleurs relatives au nerf trijumeau, en particulier les migraines ou les névralgies du trijumeau.

Les compositions pharmaceutiques selon l'invention peuvent être, à titre d'exemple, des compositions administrables par voie orale, nasale (administration par aérosol), sublinguale (administration par diffusion perlinguale), rectale, parentérale intraveineuse et percutanée. A titre d'exemple de compositions administrables par voie orale, on peut citer les comprimés, les gélules, les granules, les microsphères, les poudres et les solutions ou suspensions orales. En particulier, ils sont solubles dans les solvants du type alcool/polysorbate/eau, en particulier éthanol/Tween^{®}/eau et mannitol/eau ou à l'aide de cyclodextrines appropriées à l'administration chez l'homme, qui sont fréquemment utilisés pour l'administration par voie intraveineuse. Les compositions selon l'invention peuvent donc être administrées par voie intraveineuse. Elles peuvent également être administrées par voie orale ou nasale, en particulier via un aérosol ou par diffusion perlinguale ou au sein d'une préparation galénique appropriée (microémulsions).

Les compositions selon l'invention peuvent être administrées soit sous la forme d'aérosols (microémulsions) par voie orale ou nasale soit par voie intraveineuse. Ces voies d'administration permettent ainsi une administration de la composition selon l'invention par une voie non digestive. Ceci est particulièrement intéressant lorsque la composition comprend des composés complémentaires, qui peuvent présenter des effets non désirés sur le système digestif (en particulier l'intestin), tels que par exemple des dérivés de cannabinoïdes. Ceci permet également d'augmenter la biodisponibilité (notamment cérébrale) des composés ou associations.

De préférence, les compositions de l'invention sont appropriées pour une administration par voie orale ou par voie intraveineuse.

Dans un mode de réalisation particulier, une formulation de la présente invention comprend une cyclodextrine, telle que l'hydroxypropyl béta-cyclodextrine ou bien encore la sulfobutyl ether béta-cyclodextrine, ou du sodium de polystyrène sulfonate.

Les compositions administrées en accord avec les méthodes décrites dans la présente invention contiennent une quantité active pour une utilisation, d'un sel d'un composé de formule (I) ou d'un sel de formule (II), en particulier du composé 1. Cela signifie une quantité suffisante pour prévenir ou soulager (traiter) les douleurs relatives au nerf trijumeau, en particulier les migraines, les névralgies du trijumeau, l'algie vasculaire de la face ou les douleurs périphériques trigéminales liées à la sclérose en plaques.

La dose thérapeutique d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière.

D'après les exemples de la présente invention, l'administration d'une dose quotidienne de 4000 mg pendant 5 jours consécutifs n'a pas provoqué l'apparition d'effets secondaires indésirables. Le traitement peut donc être administré plusieurs jours sans risque de toxicité.

De manière avantageuse, les compositions selon l'invention pour leur utilisation en vue de prévenir ou traiter les douleurs liées au nerf trijumeau, en particulier les migraines et les névralgies du trijumeau, peuvent comprendre un second principe actif déjà connu pour la prévention et le traitement des douleurs.

Un autre objet de l'invention est un kit comprenant :
i) Une première composition comprenant au moins un composé de formule (I) tel que défini précédemment, et
ii) Une seconde composition comprenant au moins un second principe actif utile pour prévenir ou traiter les douleurs liées au nerf trijumeau en tant que produit de combinaison pour une utilisation simultanée, séparée ou étalée dans le temps.

Selon un mode de réalisation, le sel pharmaceutiquement acceptable d'un composé de formule (I) ou d'un sel de formule (II) selon l'invention, en particulier du composé 1, est utilisé pour la prévention des crises migraineuses.

La prévention des migraines chroniques par le sel pharmaceutiquement acceptable d'un composé de formule (I) ou d'un sel de formule (II) selon l'invention, en particulier du composé 1, est particulièrement adapté aux patients sensibles aux migraines, dont les maux de têtes sont récurrents de manière périodique ou sont provoqués par un facteur spécifique tel que la fatigue, le stress, les menstruations, la lumière vive ou artificielle (lors d'une longue exposition à des écrans par exemple), le bruit, certaines odeurs, certains aliments (alcool). Le sel pharmaceutiquement acceptable d'un composé de formule (I) peut également être utilisé de manière préventive lors de l'apparition de signes avant-coureurs d'une crise migraineuse tels qu'une aura, un trouble visuel qui peut se manifester sous forme d'une tâche sombre entourée d'un halo scintillant, de lignes géométriques, de tâches lumineuses et brillantes dans la moitié du champ de vision, d'une amputation du champ visuel, d'un dédoublement de la vue ou encore de la perte de vision temporaire d'un œil ou des deux yeux. Le patient saura identifié, par l'expérience, quel sont les facteurs à risque fortement susceptibles de lui provoquer des migraines.

Selon un mode de réalisation, les compositions pharmaceutiques selon l'invention sont utiles pour la prévention de crises migraineuses, en particulier des crises migraineuses déclenchées par un facteur à risque spécifique tel que décrit ci-dessus.

Ainsi, de manière préférée, les compositions pharmaceutiques utilisées en prévention des crises migraineuses sont administrées par voie orale. Avantageusement, ces compositions sont administrées de manière répétée.

Avantageusement, une dose thérapeutique d'un sel d'un composé de formule (I) ou d'un sel de formule (II), en particulier du composé 1 telle que décrite ci-dessus est administrée à un patient afin de prévenir les crises migraineuses. De manière préférée, ladite dose est administrée en prévention des crises migraineuses à des patients sensibles aux crises de migraines tels que décrites ci-dessus. Ainsi, en prévention des crises migraineuses, la dose thérapeutique est administrée avant l'apparition de la douleur et/ou avant l'exposition à un facteur spécifique déclencheur de la migraine. Par exemple, une femme régulièrement sujette à des migraines lors de sa période de menstruation pourra prendre la dose thérapeutique de 1 à plusieurs jours avant le début de ses règles.

De manière préférée, afin de prévenir les crises migraineuses, la dose thérapeutique administrée au patient en ayant besoin est administrée de manière répétée. Avantageusement, la dose thérapeutique est administrée en prévention des crises migraineuses par voie orale ou parentérale, avantageusement par voie orale, en particulier sous forme galénique.

Les compositions de l'invention peuvent avantageusement comprendre un second principe actif utile pour prévenir les crises migraineuses, notamment les béta-bloquants, les antidépresseurs, les anticonvulsivants, les antagonistes des canaux calciques, et/ou les inhibiteurs de l'action du CGRP.

Ainsi, afin de prévenir les crises migraineuses, la composition comprend en outre un béta-bloquant, tel l'atenolol, le metoprolol, le nadolol, le propranolol et/ou le timolol. Les antidépresseurs peuvent être choisis parmi l'amitriptyline, la doxepin, la nortriptyline, la protriptyline et/ou la venlafaxine. Les anticonvulsivants peuvent être choisis parmi la carbamazepine, la gabapentine, la topiramate et/ou le valproate/divalproex. Les antagonistes des canaux calciques peuvent être choisis parmi le verapamil, les dihydropyridines et/ou le flunarizine. Des inhibiteurs de l'action du CGRP (pour « peptide associé au gène de la calcitonine », « calcitonin gene-related peptide » en anglais) sont notamment décrits dans Tso AR et al., Curr Treat Options Neurol (2017),19 : 27 DOI 10.1007/s11940-017-0463-4 « Anti-CGRP monoclonal antibodies : the next era of migraine prevention ? »,

Selon un mode de réalisation particulier, la composition de l'invention utilisée afin de prévenir les crises migraineuses, comprend en outre un second principe actif choisi parmi les béta-bloquants, les antidépresseurs, les anticonvulsivants, les antagonistes des canaux calciques, et/ou les inhibiteurs de l'action du CGRP, avantageusement le second principe actif est choisi parmi le propranolol, le topimarate ou l'amitriptyline.

Un autre objet de l'invention est un kit comprenant :
i) une première composition comprenant au moins un composé de formule (I) tel que défini précédemment, et
ii) une seconde composition comprenant au moins un second principe actif utile pour prévenir les crises migraineuses, notamment choisi parmi choisi parmi les béta-bloquants, les antidépresseurs, les anticonvulsivants, les antagonistes des canaux calciques, et/ou les inhibiteurs de l'action du CGRP, avantageusement le second principe actif est choisi parmi le propranolol, le topimarate ou l'amitriptyline, en tant que produits de combinaison pour une utilisation simultanée, séparée ou étalée dans le temps.

Ce kit peut être utilisé en tant que médicament, notamment dans la prévention des crises migraineuses.

Selon un autre mode de réalisation, le sel pharmaceutiquement acceptable d'un composé de formule (I) ou d'un sel de formule (II) selon l'invention, en particulier du composé 1, est utilisé pour le traitement des douleurs relatives au nerf trijumeau, notamment des migraines ou la névralgie du trijumeau.

Avantageusement, selon le mode de réalisation précédent, le sel pharmaceutiquement acceptable d'un composé de formule (I) ou d'un sel de formule (II) selon l'invention, en particulier du composé 1, est utilisé pour le traitement des migraines aiguës.

Selon un mode de réalisation, les compositions pharmaceutiques selon l'invention sont utiles pour le traitement des douleurs relatives au nerf trijumeaux, notamment les migraines ou la névralgie du trijumeau, en particulier des migraines aiguës. Selon un mode de réalisation, une dose thérapeutique d'un sel d'un composé de formule (I) ou d'un sel de formule (II), en particulier du composé 1 telle que décrite ci-dessus est administrée à un patient afin de traiter les douleurs liées au nerf trijumeau. En particulier, cette dose est administrée à un patient afin de traiter des migraines, par exemple des migraines aiguës.

Selon le mode de réalisation précédent, la dose thérapeutique pour traiter des douleurs relatives au nerf trijumeau, notamment les migraines ou la névralgie du trijumeau, en particulier les migraines aiguës, est administrée au patient lorsque la douleur survient. Dans le cas d'une douleur aiguë, cette dose est généralement effective, c'est-à-dire qu'une dose thérapeutique unique est suffisante pour atténuer ou faire disparaître la douleur.

Avantageusement, afin de traiter les douleurs liées au nerf trijumeau, en particulier les migraines ou les névralgies du trijumeau, la dose thérapeutique d'un sel d'un composé de formule (I) ou d'un sel de formule (II), en particulier du composé 1 administrée au patient en ayant besoin est comprise entre 200 et 800 mg.

De manière préférée, afin de traiter les douleurs liées au nerf trijumeau, notamment les migraines, la dose thérapeutique telle que décrite ci-dessus est administrée en une à quatre prise(s). Cette dose thérapeutique peut être avantageusement administrée par voie parentérale ou par orale.

Selon un autre mode de réalisation, les compositions de l'invention peuvent avantageusement comprendre un second principe actif utile pour traiter la douleur relative au nerf trijumeau, en particulier la migraine, notamment la migraine aiguë, choisis parmi les antalgiques, les anti-inflammatoire non stéroïdiens (AINS), les opioïdes, les triptans, les modulateurs de GABA, les inhibiteurs des canaux sodiques Nav 1.7, les inhibiteurs de l'action du CGRP et/ou les cannabinoïdes, sans y être pour autant limités.

Ainsi, selon un mode de réalisation, la composition pour traiter les douleurs relatives au nerf trijumeau, en particulier les migraines, notamment les migraines aiguës, comprend en outre un antalgique, notamment les AINS, l'aspirine et le paracétamol.

Selon un autre mode de réalisation, la composition pour traiter les douleurs relatives au nerf trijumeau, en particulier les migraines, notamment les migraines aiguës, comprend en outre un AINS, tel que l'ibuprofène, le diclofénac, le piroxicam, le kétoprofène, l'indométhancine, l'acide acétylsalicylique, le célécoxib, le naproxène.

Selon un autre mode de réalisation, la composition pour traiter les douleurs relatives au nerf trijumeau, en particulier les migraines, notamment les migraines aiguës, comprend en outre un opioïde, notamment l'alfentanil, l'aniléridine, la buprénorphine, le butorphanol, le carfentanil, la codéine, le dextropropoxyphène, le fentanyl, l'hydrocodone, l'hydromorphone, la morphine, la nalbuphine, l'oxycodone, l'oxymorphone, la pentazocine, la mépéridine, le propoxyphène, le rémifentanil, le sufentanil, et le tramadol. Avantageusement, il s'agit de la morphine ou de ses dérivés, notamment de la morphine. En effet, la morphine est également capable de potentialiser l'effet analgésique induit par les composés selon l'invention.

Selon un autre mode de réalisation, la composition pour traiter les douleurs relatives au nerf trijumeau, en particulier les migraines, notamment les migraines aiguës, comprend en outre un triptan, notamment le sumatriptan, le naratriptan, le zolmitriptan, l'élétriptan, l'almotriptan, le frovatriptan, et/ou le rizatriptan.

Les modulateurs de GABA sont notamment la carbamazépine, l'oxcarbazépine, le baclofène, le clonazépam, la lamotrigine, la gabapentine ou la prégabaline. Avantageusement, il s'agit de la carbamazépine, la gabapentine ou de la prégabaline.

Des inhibiteurs de canaux sodiques Nav 1.7 sont notamment décrits dans Zakrzewska JM et al., Lancet Neurol (2017), 16 : 291-300 « Safety and efficacy of a Nav1.7 selective sodium channel blocker in patients with trigeminal neuralgia : a double-blind, placebo-controlled, randomised withdrawal phase 2a trial »,

Les compositions de l'invention pour traiter les douleurs relatives au nerf trijumeau, en particulier les migraines, notamment les migraines aiguës, peuvent en outre comprendre un au moins un dérivé de cannabinoïdes, en particulier le Δ⁹ THC, ou un protecteur de son métabolisme (revue Piomelli et al., TIPS (2000), 21 : 218-224). Il a en effet été constaté que la co-administration (simultanée ou étalée dans le temps) de faibles doses de dérivés de cannabinoïdes (en particulier, le Δ⁹ THC) permet de potentialiser l'effet analgésique des sels selon l'invention (sels des composés de formule (I) ou sels de formule (II)) sans induire, d'une manière significative, les effets néfastes desdits cannabinoïdes, qui par voie intraveineuse (iv) apparaissent dès 4-5 mg/kg (sédation). Au sens de la présente invention, on entend par l'expression « teneur très faibles en cannabinoïdes » des teneurs en cannabinoïdes inférieures à celles induisant lesdits effets secondaires indésirables. Au sens de la présente invention, on entend par l'expression « cannabinoïdes » leΔ⁹ THC, des agonistes du récepteur CB1 synthétiques ou des inhibiteurs de la dégradation de l'anandamide. Les cannabinoïdes introduits dans les compositions selon l'invention sont de préférence le Δ⁹ THC.

Selon un mode de réalisation particulier, la composition de l'invention pour traiter les douleurs relatives au nerf trijumeau, en particulier les migraines, notamment les migraines aiguës comprend en outre un second principe actif choisi parmi la morphine, le Δ⁹ THC, la carbamazépine, l'oxcarbazépine, le baclofène, le clonazépam, la lamotrigine, la gabapentine ou la prégabaline, les inhibiteurs des canaux sodiques Nav1.7, les inhibiteurs de l'action du CGRP, avantageusement le second principe actif est choisi parmi la morphine, le Δ⁹ THC, la gabapentine ou la prégabaline.

Un autre objet de l'invention est un kit comprenant :
i) une première composition comprenant au moins un composé de formule (I) tel que défini précédemment, et
ii) une seconde composition comprenant au moins un second principe actif utile pour traiter les douleurs relatives au nerf trijumeau, en particulier les migraines, par exemple les migraines aiguës, notamment choisi parmi les antalgiques, les anti-inflammatoires non stéroïdiens (AINS), les opioïdes, les triptans, les modulateurs de GABA et/ou les cannabinoïdes (avantageusement choisi parmi la morphine, le Δ⁹ THC, l'oxcarbamazépine, le baclofène, le clonazépam, la lamotrigine, la gabapentine ou la prégabaline les inhibiteurs des canaux sodiques Nav1.7, les inhibiteurs de l'action du CGRP, de préférence parmi la morphine, le Δ⁹ THC, la gabapentine ou la prégabaline), en tant que produits de combinaison pour une utilisation simultanée, séparée ou étalée dans le temps, dans la prévention ou le traitement des douleurs relatives au nerf trijumeau.

Ce kit peut être utilisé en tant que médicament, notamment dans le traitement des douleurs relatives au nerf trijumeau, telle la migraine, la névralgie du trijumeau ou la douleur neuropathique associée à la sclérose en plaques. De manière préférée, ce kit est utilisé pour traiter les migraines aiguës.

La présente invention concerne également une composition comprenant une quantité comprise entre 50 mg et 800 mg d'un sel d'un composé de formule (I) ou d'un sel de formule (II), en particulier du composé 1.

Selon une première variante, la présente invention concerne une composition comprenant une quantité comprise entre 200 mg et 800 mg d'un sel d'un composé de formule (I) ou d'un sel de formule (II), en particulier du composé 1.

Selon une seconde variante, la présente invention concerne également une composition comprenant une quantité comprise entre 100 mg et 400 mg d'un sel d'un composé de formule (I) ou d'un sel de formule (II), en particulier du composé 1.

Selon une troisième variante, la présente invention concerne également une composition comprenant une quantité comprise entre 50 mg et 200 mg d'un sel d'un composé de formule (I) ou d'un sel de formule (II), en particulier du composé 1.

Selon un mode de réalisation, les compositions telles que décrites ci-dessus sont utilisées pour le traitement de douleurs relatives au nerf trijumeaux. De manière préférée, ces compositions sont utilisées pour le traitement des migraines, par exemple des migraines aiguës.

Avantageusement, ces compositions peuvent être administrées au patient souffrant de douleurs liées au nerf trijumeaux, en particulier de migraines en respectant la posologie décrite ci-dessus.

### DESCRIPTION DES FIGURES

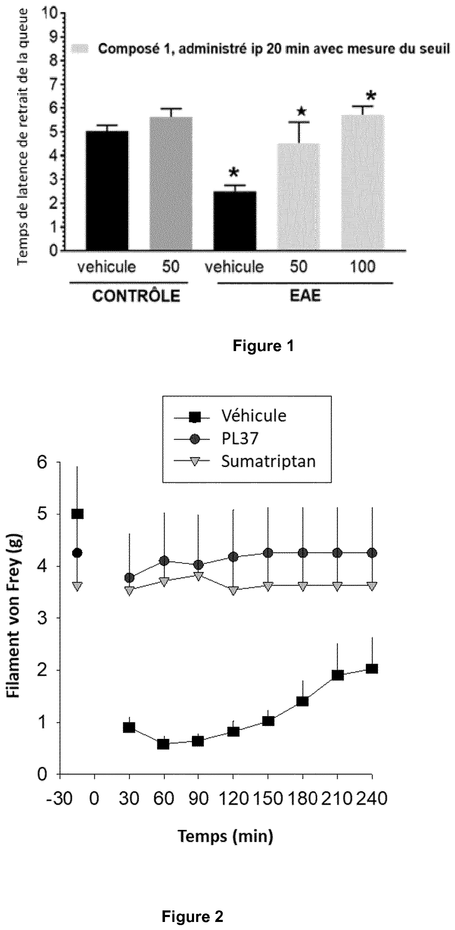

Figure 1 : Temps de latence de retrait de la queue (en secondes), pour le groupe contrôle (avec le véhicule ou 20 minutes après injection ip d'une dose de 50 mg/kg de composé 1), et pour le groupe modèle d'EAE (avec le véhicule ou 20 minutes après injection d'une dose de 50 mg/kg ou de 100 mg/kg de composé 1). Les seuils nociceptifs sont mesurés à J21, J28 et J35. La représentation est faite à J28. * : p <0.05 versus véhicule, groupe contrôle ou EAE (ANOVA + Newman-Keuls).

**Seuil Nociceptif (en secondes, Jn = Jour de mesure du seuil, 20 min après administration du composé 1 (PL37))**

| Jour | Avant Injection | Composé 1, 50 mg/kg | Composé 1, 100 mg/kg | Contrôle |
|---|---|---|---|---|
| J21 | 2,69 | 5,12(4) | | |
| J28 | 3,0/2,69 | 4,14(5) | 5,98 (5) | 4,85 (7) |
| J35 | 2,69 | | 5,46 (5) | 4,85 (7) |

Figure 2 : Céphalée/Migraine chronique (administration chronique d'ISDN) : Force de von Frey nécessaire pour entraîner un retrait de la tête (en g), en fonction du temps, mesuré en minutes, après injection iv d'une solution saline (véhicule, carré noir), d'une solution de composé 1 (rond noir) ou de sumatriptan (triangle gris).

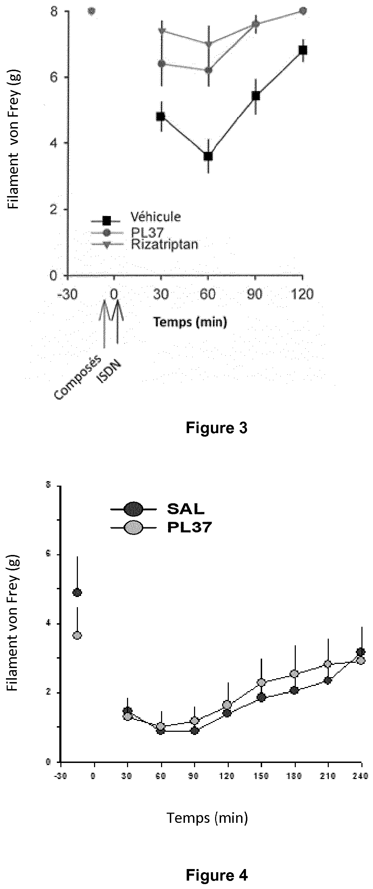

Figure 3 : Céphalée aiguë (1 administration d'ISDN) : Force de von Frey nécessaire pour entraîner un retrait de la tête (en g), en fonction du temps, mesuré en minutes, après administration orale d'une solution saline (véhicule, carré noir), d'une solution de composé 1 (rond noir) ou de rizatriptan (triangle gris).
Figure 4 : Céphalée/Migraine chronique (administration chronique d'ISDN) : Force de von Frey nécessaire pour entrainer un retrait de la tête (en g), en fonction du temps, mesuré en minutes, après administration orale d'une solution saline (véhicule, rond noir) ou de composé 1 (PL37) (rond gris, 50 mg/kg)

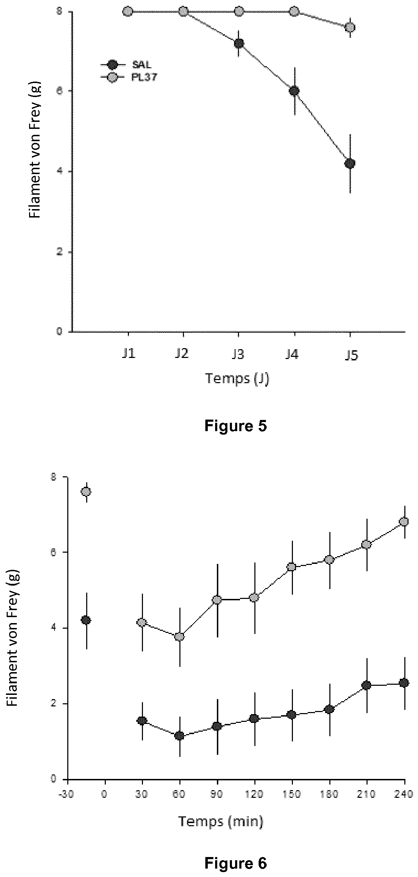

Figure 5 : Céphalée/Migraine chronique (administration chronique d'ISDN et de composé 1 (PL37)) - Sensibilité cutanée avant administration de composé 1 : Force de von Frey nécessaire pour entrainer un retrait de la tête (en g), mesurée à t=0, avant administration orale d'une solution saline (véhicule, rond noir) ou de composé 1 (rond gris, 50 mg/kg) mesurée pendant 5 jours
Figure 6 : Céphalée/Migraine chronique (administration chronique d'ISDN et de composé 1 (PL37)) : Force de von Frey nécessaire pour entrainer un retrait de la tête (en g), en fonction du temps, mesuré en minutes, après 5 jours d'administration orale d'une solution saline (véhicule, rond noir) ou de composé 1 (rond gris, 50 mg/kg) une fois par jour

### EXEMPLES

L'invention sera encore illustrée sans aucunement être limitée par les exemples ci-après.

### Exemple 1: Modèle de sclérose en plaque (encéphalite auto-immune expérimentale, EAE)

Le modèle de sclérose en plaque peut être induit chez la souris SJL, souris blanche albinos femelle, par administration d'une émulsion constituée d'un fragment de myéline et d'adjuvant de Freund complet (Aicher et al., Pain (2004), 110 : 560-570). L'inflammation du système nerveux central apparait au bout d'une dizaine de jours = encéphalomyélite auto-immune expérimentale ou EAE.

Les souris SJL femelles, achetées chez Charles-River Laboratories, arrivent âgées de 5 semaines et sont immunisées à l'âge de 6 semaines. Chaque souris reçoit, sous anesthésie au masque (isoflurane 2%), une émulsion d'un volume de 200 µL sur le flanc gauche, contant 150µg de PLP₁₃₉₋₁₅₁ (Proteolipid Protein Myelin, fragment 139-151) dans 100 µL de NaCI 0,9% stérile ainsi que 100 µL d'adjuvant incomplet de Freund (Sigma) complété par *Mycobacterium tuberculosis* (Difco, USA) inactivé, à 4 mg/mL = adjuvant complet.

Les symptômes de la maladie se déclarent entre J10 et J15, ce qui se traduit par une phase aigüe, durant laquelle les scores sont élevés et les animaux présentent une perte de sensibilité douloureuse. Cette phase précède une phase chronique qui s'installe à partir de J18 et se caractérise par une stabilisation de l'état général de l'animal et l'apparition d'un abaissement significatif du seuil de la perception douloureuse (état hyperalgique) (Aicher et al., Pain (2004), 110 : 560-570).

Les seuils nociceptifs ont été déterminés à partir de J12 par le test de l'immersion de la queue.

Le composé 1 est administré par voie ip (intrapéritonéale) à 50 mg/kg et 100 mg/kg dans un véhicule EtOH/Tween80/Eau (10/10/80) (100µL pour 10g de souris). La mesure des temps de retrait de la queue dans un bain à 48°C sont effectuées à 20 min après injection (cut-off = 10 sec).

L'administration du composé 1 (PL37) à 50 et 100 mg/kg ip permet de diminuer l'hyperalgésie chez des souris dans un modèle de sclérose en plaques/encéphalomyélite auto-immune expérimentale ou EAE (voir figure 1).

### Exemple 2 : Modèle de crise de migraine

La migraine est un trouble neurovasculaire caractérisé par des crises récurrentes de céphalées accompagnées de troubles neurologiques variables dont l'allodynie cutanée céphalique. Ce symptôme est le plus fréquent chez les patients souffrant de migraine. Il affecte 60 à 80 % des patients souffrant de migraine chronique (Guy et al., Cephalalgia (2010), 30 : 881-886 ; Lovatti, Expert Rev Neurother (2009), 9 : 395-408 ; Louter et al., Brain (2013), 136 : 3489-3496). De plus, l'apparition de l'allodynie est considérée comme un facteur de risque de chronicisation de la migraine (Louter et al., Brain (2013), 136 : 3489-3496) et est également un indicateur d'un état de sensibilisation centrale (Boyer et al., Pain (2014), 155 : 1196-1205).

### a) Modèle d'allodvnie persistante induite par l'administration systémique récurrente de dinitrate d'isosorbide (migraine/céphalée chronique)

Les effets de l'administration systémique du composé 1 sur l'allodynie mécanique céphalique ont été testés chez le rat dans un modèle de migraine déclenchée par l'injection répétée systémique d'un donneur de NO, le dinitrate d'isosorbide (ISDN). En effet, la puissante action vasodilatatrice des « donneurs de NO », expliquent leur particulière propension à déclencher des céphalées chez les sujets sains et une crise de migraine chez les patients migraineux (Hansen & Olesen, Cephalalgia (2017), 37 : 11-19).

### Animaux

Les expérimentations ont été réalisées sur des rats mâles de souche Sprague-Dawley CD (200-250g, Charles-River Laboratories). Un délai minimum de 7 jours a été respecté avant toute expérimentation.

### Evaluation de la sensibilité cutanée céphalique

Les animaux ont d'abord été soumis à des sessions d'habituation destinées à reproduire les conditions d'environnement et de manipulation de l'animal par l'expérimentateur lors du test définitif.

La sensibilité mécanique de la région périorbitaire a été mesurée par le test de von Frey qui consiste à appliquer dans cette région une gamme de filaments de von Frey, calibrés pour exercer une force (exprimée en grammes) constante, pour déterminer la force (seuil) qui entraine une réaction de retrait de la tête. Les rats ont été habitués à ces tests les 5 jours précédant l'expérience pendant une heure, de façon à ce que des mesures répétées donnent des résultats reproductibles. A la fin de la période d'habituation, les seuils de sensibilité mécanique à la douleur devaient être en moyenne de 8g (Boyer et al., Pain (2014), 155 : 1196-1205; Dallel et al., Cephalalgia (2017), Jan 1:333102417714032. doi: 10.1177/0333102417714032). Ces valeurs sont peu douloureuses puisqu'elles consistent en la mesure d'un seuil avec possibilité d'échappement et n'entrainent pas de réactions autres que le retrait de la tête. Les rats ont reçu ensuite des injections successives intrapéritonéale d'ISDN (10 mg/kg) et une évaluation de la sensibilité cutanée a été réalisée à l'aide du test de von Frey. Une allodynie mécanique s'est développée progressivement, se traduisant par une diminution des valeurs de force nécessaires (seuils) pour induire le retrait de la tête. Le jour de l'expérience, les forces exercées par les filaments de von Frey et entrainant le retrait de la tête ont été mesurées sur chaque rat en condition contrôle toutes les 30 minutes pendant 4 heures.

### Evaluation de l'activité antimigraineuse

Le jour de l'expérimentation, les rats ont reçu une première injection (intraveineuse) de sérum physiologique, de sumatriptan ou de composé 1 suivie, 5 minutes après, d'une seconde injection intrapéritonéale d'ISDN (10 mg/kg). Le rat a été ensuite replacé dans la boîte d'observation. Les forces induisant le retrait de la tête ont été mesurées toutes les 30 minutes pendant 4 heures, pour suivre la cinétique de l'effet. 3 groupes ont été constitués :
- 1 groupe témoin : injection intraveineuse de sérum physiologique (n = 10)
- 1 groupe test : injection intraveineuse de composé 1 (20 mg/kg ; n = 10)
- 1 groupe référence : injection intraveineuse sumatriptan (300 µg/kg ; n = 10)

### Analyse des données

Une analyse de variance à 2 voies (effet temps et traitement) en mesures répétées a été effectuée et a été suivie d'un test post-hoc. Les résultats sont présentés sur la figure 2.

On constate que le composé 1 - administré par voie intraveineuse à la dose de 20 mg/kg - réduit considérablement la sensibilité céphalique cutanée, ce qui est significatif d'une diminution de l'activité migraineuse associée, en conditions chroniques.

### b) Modèle d'allodynie mécanique induite par l'administration unique de dinitrate d'isosorbide (migraine/céphalée aiguë)

Les effets de l'administration systémique du composé 1 sur l'allodynie mécanique céphalique ont été testés chez le rat dans un modèle de migraine déclenchée par l'injection unique systémique d'un donneur de NO, le dinitrate d'isosorbide (ISDN). En effet, la puissante action vasodilatatrice des « donneurs de NO », expliquent leur particulière propension à déclencher des céphalées chez les sujets sains et une crise de migraine chez les patients migraineux (Hansen & Olesen, Cephalalgia (2017), 37 : 11-19).

### Animaux

Les expérimentations ont été réalisées sur des rats mâles de souche Sprague-Dawley CD (200-250g, Charles-River Laboratories). Un délai minimum de 7 jours a été respecté avant toute expérimentation.

### Evaluation de la sensibilité cutanée céphalique

Les animaux ont d'abord été soumis à des sessions d'habituation destinées à reproduire les conditions d'environnement et de manipulation de l'animal par l'expérimentateur lors du test définitif.

La sensibilité mécanique de la région périorbitaire a été mesurée par le test de von Frey qui consiste à appliquer dans cette région une gamme de filaments de von Frey, calibrés pour exercer une force (exprimée en grammes) constante, pour déterminer la force (seuil) qui entraine une réaction de retrait de la tête. Les rats ont été habitués à ces tests les 5 jours précédant l'expérience pendant une heure, de façon à ce que des mesures répétées donnent des résultats reproductibles. A la fin de la période d'habituation, les seuils de sensibilité mécanique à la douleur devaient être en moyenne de 8g (Boyer et al., Pain (2014), 155 : 1196-1205; Dallel et al., Cephalalgia (2017), Jan 1:333102417714032. doi: 10.1177/0333102417714032). Ces valeurs sont peu douloureuses puisqu'elles consistent en la mesure d'un seuil avec possibilité d'échappement et n'entrainent pas de réactions autres que le retrait de la tête. Les rats ont reçu ensuite une injection intrapéritonéale d'ISDN (10 mg/kg) et une évaluation de la sensibilité cutanée a été réalisée à l'aide du test de von Frey. Une allodynie mécanique s'est développée progressivement, se traduisant par une diminution des valeurs de force nécessaires (seuils) pour induire le retrait de la tête. Le jour de l'expérience, les forces exercées par les filaments de von Frey et entrainant le retrait de la tête ont été mesurées sur chaque rat en condition contrôle toutes les 30 minutes pendant 4 heures.

### Evaluation de l'activité antimigraineuse

Le jour de l'expérimentation, les rats ont reçu une première injection (par voie orale) de sérum physiologique, de rizatriptan (10 µg/kg) ou de composé 1 (50 mg/kg) suivie, 5 minutes après, d'une injection intrapéritonéale d'ISDN (10 mg/kg). Le rat a été ensuite replacé dans la boîte d'observation. Les forces induisant le retrait de la tête ont été mesurées toutes les 30 minutes pendant 4 heures, pour suivre la cinétique de l'effet. 3 groupes ont été constitués :
- 1 groupe témoin : administration orale de sérum physiologique (n = 10)
- 1 groupe test : administration orale de composé 1 (50 mg/kg ; n = 10)
- 1 groupe référence : administration orale rizatriptan (10 µg/kg ; n = 10)

### Analyse des données

Une analyse de variance à 2 voies (effet temps et traitement) en mesures répétées a été effectuée et a été suivie d'un test post-hoc. Les résultats sont présentés sur la figure 3.

On constate que le composé 1 - administré par voie orale à la dose de 50 mg/kg - réduit considérablement la sensibilité céphalique cutanée, ce qui est significatif d'une diminution de l'intensité de la crise aigüe de migraine.

### Exemple 3 : Modèle de prévention des crises de migraine par voie orale

Les effets de l'administration orale du composé 1 sur l'allodynie mécanique céphalique ont été testés chez le rat dans un modèle de migraine chronique déclenchée par l'injection répétée systémique d'un donneur de NO, le dinitrate d'isosorbide (ISDN). En effet, la puissante action vasodilatatrice des « donneurs de NO », explique leur particulière propension à déclencher des céphalées chez les sujets sains et une crise de migraine chez les patients migraineux (Hansen & Olesen, Cephalalgia (2017), 37: 11-19).

### Animaux

Les expérimentations ont été réalisées sur des rats mâles (10 rats par groupe) de souche Sprague-Dawley CD (200-250g, Charles-River Laboratories), randomisés pour l'attribution dans chaque groupe avant expérimentation. Un délai minimum de 7 jours a été respecté avant toute expérimentation. Les expériences sont réalisées en aveugle par l'expérimentateur.

### Evaluation de la sensibilité cutanée céphalique

Les animaux ont d'abord été soumis à des sessions d'habituation destinées à reproduire les conditions d'environnement et de manipulation de l'animal par l'expérimentateur lors du test définitif.

La sensibilité mécanique de la région périorbitaire a été mesurée par le test de von Frey qui consiste à appliquer dans cette région une gamme de filaments de von Frey, calibrés pour exercer une force (exprimée en grammes) constante, pour déterminer la force (seuil) qui entraine une réaction de retrait de la tête. Les rats ont été habitués à ces tests les 5 jours précédant l'expérience pendant une heure, de façon à ce que des mesures répétées donnent des résultats reproductibles. A la fin de la période d'habituation, les seuils de sensibilité mécanique à la douleur devaient être en moyenne de 8 g (Boyer et al., Pain (2014), 155 : 1196-1205*;* Dallel et al., Cephalalgia (2017), Jan 1:333102417714032. doi: 10.1177/0333102417714032*).* Ces valeurs sont peu douloureuses puisqu'elles consistent en la mesure d'un seuil avec possibilité d'échappement et n'entrainent pas de réactions autres que le retrait de la tête. Les rats ont reçu ensuite des injections successives intrapéritonéale d'ISDN (10 mg/kg) et une évaluation de la sensibilité cutanée a été réalisée à l'aide du test de von Frey. Une allodynie mécanique s'est développée progressivement, se traduisant par une diminution des valeurs de force nécessaires (seuils) pour induire le retrait de la tête.

La sensibilité mécanique cutanée a été mesurée avant et le jour du test (Figure 4, cas d'une simple administration de composé 1 ou Figure 6, cas d'une administration répétée de composé 1) ou avant et le jour du test (Jours 1, 2, 3, 4 et 5) (Figure 5, administration répétée de composé 1) avant injection et à des intervalles de 30 min pendant 4h dans les différents groupes de rats recevant intrapéritonéale l'ISDN (10 mg/kg, volume 10 mL/kg).

### Evaluation de l'activité antimigraineuse

Le jour de l'expérimentation, les rats ont reçu une première administration orale de véhicule (10% EtOH dans sérum physiologique NaCl 0.9%) ou de composé 1 (50 mg/kg dans le véhicule) suivie, 5 minutes après, d'une seconde injection intrapéritonéale d'ISDN (10 mg/kg). Le rat a été ensuite replacé dans la boîte d'observation. Les forces induisant le retrait de la tête ont été mesurées toutes les 30 minutes pendant 4 heures, pour suivre la cinétique de l'effet.

### Analyse des données

Une analyse de variance à 2 voies (effet temps et traitement) en mesures répétées a été effectuée et a été suivie d'un test post-hoc.

Le composé 1 - administré par voie orale à la dose de 50 mg/kg, administré 1 fois le jour de l'expérience (Figure 4) n'a pas d'effet sur la sensibilité céphalique cutanée. Le composé 1, administré de manière répétée sur plusieurs jours (5 jours) réduit considérablement la sensibilité céphalique cutanée par rapport au véhicule un jour par rapport au précédent, avant administration d'ISDN (Figure 5) ou en fonction du temps, après administration d'ISDN (Figure 6), ce qui est significatif d'une prévention et de la diminution de l'activité migraineuse associée, en condition chronique.

### Exemple 4 : Posologie du composé 1 - Etude des effets indésirables observés durant la phase 1b de l'étude clinique du composé 1 (PL37)

### Détails sur les conditions de l'expérience

Quarante sujets volontaires sains âgés de 18 à 65 ans divisés en cinq groupes de huit sujets (6 recevant selon un tirage au sort quatre doses quotidiennes de composé 1 (PL37), deux recevant quatre doses quotidiennes de placebo).

Les doses indiquées dans le tableau suivant ont été administrées 4 fois par jours pendant 5 jours, la dose journalière variant de 800 à 4000 mg, la quantité totale sur cinq jours étant comprise entre 4000 et 20000 mg.

| | Composé 1 (PL37) | | | | | Placebo | Total |
|---|---|---|---|---|---|---|---|
| | 200mg | 400mg | 600mg | 800mg | 1000mg | | |
| | N=6 | N=6 | N=6 | N=6 | N=6 | N=10 | N=40 |
| | n(%)E | n(%)E | n(%)E | n(%)E | n(%)E | n(%)E | n(%)E |
| Sujets avec : | | | | | | | |
| | 1(16.7) | 5(83.3)1 | 4(66.7) | 6(100.0)1 | 4(66.7)1 | 5(50.0)1 | 25(62.5)57 |
| EIACTs | 3 | 2 | 7 | 0 | 5 | 0 | |
| EILs | 0 | 0 | 0 | 0 | 0 | 0 | 0 |
| EIGs | 0 | 0 | 0 | 0 | 0 | 0 | 0 |
| Décès | 0 | 0 | 0 | 0 | 0 | 0 | 0 |

| | | | | | | | |
|---|---|---|---|---|---|---|---|
| E= nombre d'effets indésirables n=nombre de sujets ayant subit des effets indésirables N= nombre de sujets par groupe de traitement EILs= effets indésirable limitant EIACTs=effets indésirables apparus au cours du traitement EIGs=effets indésirables graves | | | | | | | |

## Revendications

1. Sel pharmaceutiquement acceptable, en particulier sel d'addition d'acide, d'un composé de formule (I), pour utilisation pour la prévention ou le traitement des douleurs relatives au nerf trijumeau :
H₂N-CH(R₁)-CH₂-S-S-CH₂-CH(R₂)-CONH-CH(R₃)-COOR₄ (I)
dans laquelle :
∘ R₁ représente :
- une chaîne hydrocarbonée, saturée ou insaturée, linéaire ou ramifiée, comportant de 1 à 6 atomes de carbones, éventuellement substituée par :
* un radical OR, SR ou S(O)R, dans chacun de ces radicaux R représente un hydrogène, une chaîne hydrocarbonée linéaire ou ramifiée de 1 à 4 atomes de carbone, un radical phényle ou benzyle,
* un radical phényle ou benzyle,
- un radical phényle ou benzyle éventuellement substitué par :
* 1 à 5 halogènes, notamment le fluor,
* un radical OR, SR ou S(O)R, dans chacun de ces radicaux R ayant la même signification que précédemment,
- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde ;
∘ R₂ représente :
- un radical phényle ou benzyle, éventuellement substitué par :
* 1 à 5 atomes d'halogènes notamment le fluor,
* un radical OR ou SR, dans chacun de ces radicaux R ayant la même signification que précédemment,
* un groupement amino éventuellement mono- ou di-substitué par un groupement aliphatique, cyclique ou linéaire, de 1 à 6 atomes de carbones,
* un cycle aromatique à 5 ou 6 atomes,
- un hétérocycle aromatique à 5 ou 6 atomes, l'hétéroatome étant un oxygène, un azote ou un soufre,
- un groupe méthylène substitué par un hétérocycle à 5 ou 6 atomes, aromatique ou saturé, l'hétéroatome étant un oxygène, un azote ou un soufre, les atomes d'azote et de soufre pouvant être oxydés sous forme de N-oxyde ou de S-oxyde ;
∘ R₃ représente :
- un hydrogène,
- un groupement OH ou OR, R ayant la même signification que précédemment,
- une chaîne hydrocarbonée saturée (alkyle), linéaire ou ramifiée, comportant de 1 à 6 atomes de carbone, éventuellement substitué par un radical OR ou SR, dans chacun de ces radicaux R a la même signification que précédemment,
- un radical phényle ou benzyle, éventuellement substitué par :
* 1 à 5 halogènes notamment le fluor,
* un radical OR ou SR, R ayant la même définition que précédemment. et
∘ OR₄ représente
- un radical glycolate OCH₂COOR' ou lactate OCH(CH₃)COOR', dans chacun de ces radicaux R' représente :
• une alkyle en C₁-C₆, linéaire ou ramifié éventuellement substitué par un groupement alkoxy en C₁-C₃, de préférence un groupe alkyle en C₁-C₄ éventuellement substitué par un groupe méthoxy,
• un groupement cycloalkyle de C₅-C₈, de préférence un groupe cycloalkyle en C₅-C₆,
• un groupement phényle, benzyle, hétéroaryle, alkylhétéroaryle ;
- un groupement OCH(R")O(CO)OR' ou OCH(R")O(CO)R', dans chacun de ces radicaux R' a la même signification que précédemment et R" représente :
• un atome d'hydrogène,
• une chaîne alkyle en C₁-C₆ linéaire ou ramifiée éventuellement substituée par un groupement alkoxy de C₁-C₃, de préférence un groupe alkyle en C₁-C₄ éventuellement substitué par un groupe méthoxy,
• un groupe cycloalkyle de C₅-C₈, de préférence un groupe cycloalkyle en C₅-C₆,
• un groupement phényle, benzyle, hétéroaryle, alkylhétéroaryle ;
- un radical triglycéride OCH(CH₂OCOR')₂ ou OCH₂-CH(OCOR')-CH₂OCOR', dans chacun de ces radicaux R' ayant la même signification que précédemment ;
- un radical glycoside tel que D-glucose, β-D-glucopyranose, α- ou β-galactopyranose ;
- un radical sulfonate OCH₂CH₂(SO₂)CH_{3;}
- un radical OCH(CH₂OH)₂.

2. Sel pour utilisation selon la revendication 1, **caractérisé en ce que** R₁ représente un radical alkyle ayant de 1 à 4 atomes de carbone substitué par un radical SR, R étant tel que défini dans la revendication 1, et représentant en particulier une chaîne hydrocarbonée saturée linéaire ou ramifiée de 1 à 4 atomes de carbone.

3. Sel pour utilisation selon la revendication 1 ou 2, **caractérisé en ce que** R₂ représente un radical benzyle ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde.

4. Sel pour utilisation selon l'une quelconque des revendications 1 à 3, **caractérisé en ce que** R₃ représente un atome d'hydrogène ou un radical alkyle ayant de 1 à 6 atomes de carbone substitué par un radical OH ou SH.

5. Sel pour utilisation selon l'une quelconque des revendications 1 à 4, **caractérisé en ce que** OR₄ représente un groupement OCH(R")O(CO)OR' ou OCH(R")O(CO)R', le radical R' représentant une chaîne alkyle en C₁-C₄, et le radical R" représentant un radical méthyle, CH(CH₃)₂, cyclohexyle ou phényle.

6. Sel pour utilisation selon l'une quelconque des revendications 1 à 5, **caractérisé en ce que** le composé de formule (I) est sous forme de sel de fumarate.

7. Sel pour utilisation selon l'une quelconque des revendications 1 à 5, **caractérisé en ce qu'**il répond à la formule (II) suivante :
A⁻, H₃N⁺-CH(R₁)-CH₂-S-S-CH₂-CH(R₂)-CONH-CH(R₃)-COOR₄ (II)
dans laquelle :
∘ A⁻ représente un anion phosphate, chlorure, acétate, méthanesulfonate, borate, lactate, fumarate, succinate, hémisuccinate, citrate, tartrate, hémitartrate, maléate, ascorbate, hemifumarate, hexanoate, heptanoate, hippurate, hydrocinnamate, phénylglyoxylate ou nicotinate, de préférence A⁻ représente un anion fumarate ;
∘ R₁ représente une chaîne hydrocarbonée saturée ou insaturée, linéaire ou ramifiée comportant de 1 à 6 atomes de carbones, substituée ou non par un groupement OR, SR ou S(O)R dans lequel R représente un hydrogène, une chaîne hydrocarbonée linéaire ou ramifiée de 1 à 4 carbones, un radical phényle ou benzyle ;
∘ R₂ représente :
- un groupe phényle ou benzyle, éventuellement substitué par :
*1 à 5 atomes d'halogènes notamment le fluor, un hydroxyle ou un thiol, un éther OR ou un thio-ether SR, R ayant la même signification que ci-dessus,
*un cycle aromatique ou hétérocyclique aromatique à 5 ou 6 atomes, l'hétéroatome étant un oxygène, un azote ou un soufre,
- un groupe méthylène substitué par un hétérocycle à 5 ou 6 atomes, aromatique ou saturé, l'hétéroatome étant un oxygène, un azote ou un soufre, les atomes d'azote et de soufre étant éventuellement oxydés sous forme de N-oxyde ou de S-oxyde ;
∘ R₃ représente :
- un hydrogène,
- un groupement OH ou OR, R ayant la même signification que ci-dessus,
- un alkyle en C₁-C₆ linéaire ou ramifié, éventuellement substitué par un groupe OH, OR, SH ou SR, R ayant la même signification que ci-dessus,
- un groupe phényle ou benzyle substitué ou non par 1 à 5 halogènes notamment le fluor, par un groupe OR ou SR, R ayant la même définition que ci-dessus ;
∘ OR₄ représente :
- un radical glycolate OCH₂COOR' ou lactate OCH(CH₃)COOR',
- un groupement OCH₂OCOR' ou OCH(CH₃)OCOOR', ou
- un radical triglycéride OCH(CH₂OCOR')₂ ou OCH₂-CH(OCOR')-CH₂OCOR',
R' représentant un alkyle en C₁-C₄ linéaire ou ramifié.

8. Sel pour utilisation selon la revendication 1, **caractérisé en ce qu'**il est choisi parmi les sels pharmaceutiquement acceptables des composés suivants :
1-(2-(1-(2,3-diacétoxypropoxycarbonyl)-éthylcarbamoyl)-3-thiophèn-3-yl propyl disulfanylméthyl)-3-méthylsulfanylpropyl-amine,
1-(2-(1-(2-méthanesulfonyléthoxycarbonyl)-éthylcarbamoyl)-3-thiophèn-3-ylpropyl disulfanylméthyl)-3-méthylsulfanylpropyl- amine,
1-(2-(1-(1-éthoxycarbonyloxyéthoxycarbonyl))-éthylcarbamoyl)-3-thiophèn-3-yl-propyl disulfanylméthyl)-3-méthylsulfanylpropyl- amine,
1-(2-(1-éthoxycarbonylméthyloxycarbonyléthylcarbamoyl)-3-thiophèn-3-yl-propyl disulfanylméthyl)-3-méthylsulfanylpropyl- amine,
1-(2-(1-(1-éthoxycarbonyloxyéthoxycarbonyl)-2-hydroxypropylcarbamoyl)-3-thiophèn-3-ylpropyldisulfanylméthyl)-3-méthylsulfanylpropyl- amine,
1-(2-(1-(2-acétoxy-1-acétoxyméthyléthoxycarbonyl)-éthylcarbamoyl)-3-thiophèn-3-yl propyldisulfanylméthyl)-3-méthylsulfanylpropyl- amine,
1-(2-(1-(2-hydroxy-1-hydroxyméthyléthoxycarbonyl)-éthylcarbamoyl)-3-thiophèn-3-yl propyldisulfanylméthyl)-3-méthylsulfanylpropyl- amine,
1-(2-(1-(3,4,5,6-tétrahydroxytétrahydropyran-2-ylméthoxycarbonyl)-éthylcarbamoyl)-3-thiophèn-3-yl-propyldisulfanylméthyl)-3-méthylsulfanylpropyl-amine,
1-(2-(1-(1-éthoxycarbonyloxy-éthoxycarbonyl)-2-hydroxypropylcarbamoyl)-3-phényl propyldisulfanylméthyl)-3-méthylsulfanylpropyl- amine,
1-(2-(1-(2-acétoxy-1-acétoxyméthyl-éthoxycarbonyl)-2-hydroxypropylcarbamoyl)-3-phénylpropyldisulfanylméthyl)-3-méthylsulfanylpropyl-amine,
1-(2-((1-éthoxycarbonyloxy-éthoxycarbonylméthyl)-carbamoyl)-3-phényl-propyl disulfanylméthyl)-3-méthylsulfanylpropyl- amine,
3-(2-amino-4-méthylsulfanyl-butyldisulfanyl)-2-benzyl-N-(5-éthyl-(1,3,4)-thiadiazol-2-yl)-propionamide,
1-(2-((1-éthoxycarbonyloxy-2-méthyl-propoxycarbonylméthyl)-carbamoyl)-3-phényl-propyldisulfanylméthyl)-3-méthylsulfanyl-propyl-amine,
1-(2-((cyclohexyl-éthoxycarbonyloxy-méthoxycarbonylméthyl)-carbamoyl)-3-phényl-propyldisulfanylméthyl)-3-méthylsulfanyl-propyl-amine,
1-(2-((éthoxycarbonyloxy-phényl-méthoxycarbonylméthyl)-carbamoyl)-3-phényl-propyldisulfanylméthyl)-3-méthylsulfanyl-propyl-amine,
3-méthylsulfanyl-1-(3-phényl-2-((1-propionyloxy-éthoxycarbonylméthyl)-carbamoyl)-propyldisulfanylméthyl)-propyl-amine,
1-(2-((2-méthyl-1-propionyloxy-propoxycarbonylméthyl)-carbamoyl)-3-phényl-propyldisulfanylméthyl)-3-méthylsulfanyl-propyl-amine,
1-(2-((cyclohexyl-propionyloxy-méthoxycarbonylméthyl)-carbamoyl)-3-phényl-propyldisulfanylméthyl)-3-méthylsulfanyl-propyl-amine, 3-méthylsulfanyl-1-(3-phényl-2-((phényl-propionyloxy-méthoxycarbonylméthyl)-carbamoyl)-propyl disulfanylméthyl)-propyl-amine.

9. Sel pour utilisation selon la revendication 1, **caractérisé en ce qu'**il s'agit du (5S, 10S)-10-benzyl-16-methyl-11,14,18-trioxo-15,17,19-trioxa-2,7,8-trithia-12-azahenicosan-5-aminium fumarate.

10. Sel pour utilisation selon l'une quelconque des revendications 1 à 9, **caractérisé en ce qu'**une dose thérapeutique dudit sel est administrée au patient en ayant besoin en une à quatre prise(s).

11. Sel pour utilisation selon l'une quelconque des revendications 1 à 10, **caractérisée en ce qu'**une dose thérapeutique dudit sel est administrée au patient en ayant besoin pour le traitement des douleurs relatives au nerf trijumeau, en particulier les migraines ou les névralgies du trijumeau, ladite dose étant comprise entre 200 et 800 mg.

12. Sel pour utilisation selon l'une quelconque des revendications 1 à 10, **caractérisé en ce que** le sel est utilisé pour la prévention des crises migraineuses.

13. Composition pour utilisation pour la prévention ou le traitement des douleurs relatives au nerf trijumeau, comprenant à titre de principe actif au moins un sel pharmaceutiquement acceptable d'un composé de formule (I) tel que défini dans l'une quelconque des revendications 1 à 6, 8 et 9 ou au moins un sel de formule (II) tel que défini dans la revendication 7, et au moins un excipient pharmaceutiquement acceptable.

14. Composition pour utilisation selon la revendication 13, **caractérisée en ce qu'**elle comprend en outre un second principe actif utile pour prévenir ou traiter les douleurs relatives au nerf trijumeau.

15. Kit comprenant :
i) une première composition comprenant au moins un sel pharmaceutiquement acceptable d'un composé de formule (I) tel que défini précédemment, et
ii) une seconde composition comprenant au moins un second principe actif utile pour traiter la douleur relative au nerf trijumeau, en tant que produits de combinaison pour une utilisation simultanée, séparée ou étalée dans le temps, dans la prévention ou le traitement des douleurs relatives au nerf trijumeau, telles les migraines ou les névralgies du trijumeau.

16. Composition selon la revendication 14 ou kit selon la revendication 15 pour utilisation pour la prévention des douleurs relatives au nerf trijumeau, **caractérisé en ce que** le second principe actif est choisi parmi les béta-bloquants, les antidépresseurs, les anticonvulsivants, les antagonistes des canaux calciques, et/ou les inhibiteurs de l'action du CGRP, de préférence il est choisi parmi le propranolol, l'amitriptyline et/ou le topiramate.

17. Composition selon la revendication 14 ou kit selon la revendication 15 pour utilisation pour le traitement des douleurs relatives au nerf trijumeau, **caractérisé en ce que** le second principe actif est choisi parmi les antalgiques, les anti-inflammatoires non stéroïdiens (AINS), les opioïdes, les triptans, les modulateurs de GABA, les inhibiteurs des canaux sodiques Nav 1.7, les inhibiteurs de l'action du CGRP et/ou les cannabinoïdes, de préférence il est choisi parmi la morphine, le Δ⁹ THC, la Copie propre
carbamazépine, l'oxcarbazépine, le baclofène, le clonazépam, la lamotrigine, la gabapentine ou la prégabaline.

18. Composé selon l'une quelconque des revendications 1 à 12, ou composition selon l'une quelconque des revendications 13, 14 ou 16, ou kit selon l'une quelconque des revendications 15 ou 16, pour utilisation pour prévenir les migraines ou les névralgies du trijumeau.

19. Composé selon l'une quelconque des revendications 1 à 12, ou composition selon l'une quelconque des revendications 13, 14 ou 17 ou kit selon l'une quelconque des revendications 15 ou 18, pour utilisation pour traiter les migraines ou les névralgies du trijumeau.

20. Composé selon l'une quelconque des revendications 1 à 12, ou composition selon l'une quelconque des revendications 13, 14 ou 16, ou kit selon l'une quelconque des revendications 15 ou 16, pour utilisation pour prévenir les névralgies essentielles du trijumeau, les névralgies symptomatiques du trijumeau, les migraines, l'allodynie cutanée céphalique, l'algie vasculaire de la face, ou les douleurs périphériques trigéminales associées à la sclérose en plaques.

21. Composé selon l'une quelconque des revendications 1 à 12, ou composition selon l'une quelconque des revendications 13, 14 ou 17, ou kit selon l'une quelconque des revendications 15 ou 18, pour utilisation pour traiter les névralgies essentielles du trijumeau, les névralgies symptomatiques du trijumeau, les migraines, l'allodynie cutanée céphalique, l'algie vasculaire de la face, ou les douleurs périphériques trigéminales associées à la sclérose en plaques.

22. Composition comprenant 50 à 800 mg d'un sel tel que défini dans les revendications 1 à 12, un excipient pharmaceutiquement acceptable et un second principe actif, ledit second principe actif étant utile pour prévenir ou traiter les douleurs relatives au nerf trijumeau.

## Patentansprüche

1. Pharmazeutisch akzeptables Salz, vor allem Säureadditionssalz, einer Verbindung der Formel (I), zur Verwendung zwecks Vorbeugung oder Behandlung der Schmerzen, die sich auf den Nervus trigeminus beziehen:
H₂N-CH(R₁)-CH₂-S-S-CH₂-CH(R₂)-CONH-CH(R₃)-COOR₄ (I)
in welcher:
∘ R₁ darstellt:
- eine gesättigte oder ungesättigte, lineare oder verzweigte Kohlenwasserstoffkette mit 1 bis 6 Kohlenstoffatomen, eventuell substituiert durch:
* ein OR-, SR- oder S(O)R-Radikal, wobei in jedem dieser Radikale R einen Wasserstoff, eine lineare oder verzweigte Kohlenwasserstoffkette aus 1 bis 4 Kohlenstoffatomen, ein Phenyl- oder Benzylradikal darstellt,
* ein Phenyl- oder Benzylradikal,
- ein Phenyl- oder Benzylradikal, eventuell substituiert durch:
* 1 bis 5 Halogene, insbesondere Fluor,
* ein OR-, SR- oder S(O)R-Radikal, wobei in jedem dieser Radikale R dieselbe Bedeutung hat wie oben,
- ein Methylenradikal, substituiert durch einen aromatischen oder gesättigten Heterozyklus aus 5 oder 6 Atomen, der als Heteroatom ein Stickstoff- oder Schwefelatom besitzt, eventuell oxidiert in Form von N-Oxid oder von S-Oxid;
∘ R₂ darstellt:
- ein Phenyl- oder Benzylradikal, eventuell substituiert durch:
* 1 bis 5 Halogenatome, insbesondere Fluor,
* ein OR- oder SR-Radial, wobei in jedem dieser Radikale R dieselbe Bedeutung hat wie oben,
* eine Aminogruppe, eventuell mono- oder disubstituiert durch eine aliphatische, zyklische oder lineare Gruppe aus 1 bis 6 Kohlenstoffatomen,
* einen aromatischen Ring aus 5 oder 6 Atomen,
- einen aromatischen Heterozyklus aus 5 oder 6 Atomen, wobei das Heteroatom ein Sauerstoff, ein Stickstoff oder ein Schwefel ist,
- eine Methylengruppe, substituiert durch einen aromatischen oder gesättigten Heterozyklus aus 5 oder 6 Atomen, wobei das Heteroatom ein Sauerstoff, ein Stickstoff oder ein Schwefel ist, wobei die Stickstoff- und Schwefelatome in Form von N-Oxid oder von S-Oxid oxidiert sein können;
∘ R₃ darstellt:
- ein Wasserstoff,
- eine OH- oder OR-Gruppe, wobei R dieselbe Bedeutung hat wie oben,
- eine gesättigte (Alkyl), lineare oder verzweigte Kohlenwasserstoffkette mit 1 bis 6 Kohlenstoffatomen, eventuell substituiert durch ein OR- oder SR-Radikal, wobei in jedem dieser Radikale R dieselbe Bedeutung hat wie oben,
- ein Phenyl- oder Benzylradikal, eventuell substituiert durch:
* 1 bis 5 Halogene insbesondere Fluor,
* ein OR- oder SR- Radikal, wobei R dieselbe Definition hat wie oben, und
∘ OR₄ darstellt
- ein Glyoclatradikal OCH₂COOR' oder Laktatradikal OCH(CH₃)COOR', wobei in jedem dieser Radikale R' darstellt:
• ein lineares oder verzweigtes C₁-C₆-Alkyl, eventuell substituiert durch eine C₁-C₃-Alkoxygruppe, vorzugsweise eine C₁-C₄-Alkylgruppe, eventuell substituiert durch eine Methoxygruppe,
• eine C₅-C₈-Cycloalkylgruppe, vorzugsweise eine C₅-C₆-Cycloalkylgruppe,
• eine Phenyl-, Benzyl-, Heteroaryl-, Alkylheteroarylgruppe;
- eine Gruppe OCH(R")O(CO)OR' oder OCH(R")O(CO)R', wobei in jedem dieser Radikale R' dieselbe Bedeutung hat wie oben und R" darstellt:
• ein Wasserstoffatom,
• eine lineare oder verzweigte C₁-C₆-Alkylkette, eventuell substituiert durch eine C₁-C₃-Alkoxygruppe, vorzugsweise eine C₁-C₄-Alkylgruppe, eventuell substituiert durch eine Methoxygruppe,
• eine C₅-C₈-Cycloalkylgruppe, vorzugsweise eine C₅-C₆-Cycloalkylgruppe,
• eine Phenyl-, Benzyl-, Heteroaryl-, Alkylheteroarylgruppe;
- ein Triclyceridradikal OCH(CH₂OCOR')₂ oder OCH₂-CH(OCOR')-CH₂OCOR', wobei in jedem dieser Radikale R' dieselbe Bedeutung hat wie oben;
- ein Glycosidradikal wie D-Glucose, β-D-Glucopyranose, α- oder β-Galactopyranose;
- ein Sulfonatradikal OCH₂CH₂(SO₂)CH₃;
- ein Radikal OCH(CH₂OH)₂.

2. Salz zur Verwendung nach Anspruch 1, **dadurch gekennzeichnet, dass** R₁ ein Alkylradikal mit 1 bis 4 Kohlenstoffatomen darstellt, substituiert durch ein Radikal SR, wobei R wie in Anspruch 1 definiert ist, und vor allem eine gesättigte lineare oder verzweigte Kohlenwasserstoffkette aus 1 bis 4 Kohlenstoffatomen darstellt.

3. Salz zur Verwendung nach Anspruch 1 oder 2, **dadurch gekennzeichnet, dass** R₂ ein Benzylradikal oder ein Methylenradikal darstellt, substituiert durch einen aromatischen oder gesättigten Heterozyklus aus 5 oder 6 Atomen, der als Heteroatom ein Stickstoff- oder Schwefelatom besitzt, eventuell oxidiert in Form von N-Oxid oder von S-Oxid.

4. Salz zur Verwendung nach einem der Ansprüche 1 bis 3, **dadurch gekennzeichnet, dass** R₃ ein Wasserstoffatom oder ein Alkylradikal mit 1 bis 6 Kohlenstoffatomen darstellt, substituiert durch ein OH- oder SH-Radikal.

5. Salz zur Verwendung nach einem der Ansprüche 1 bis 4, **dadurch gekennzeichnet, dass** OR₄ eine Gruppe OCH(R")O(CO)OR' oder OCH(R")O(CO)R' darstellt, wobei das Radikal R' eine C₁-C₄-Alkylkette darstellt und das Radikal R" ein Methyl-, CH(CH₃)₂-, Cyclohexyl- oder Phenylradikal darstellt.

6. Salz zur Verwendung nach einem der Ansprüche 1 bis 5, **dadurch gekennzeichnet, dass** die Verbindung der Formel (I) in Form von Fumaratsalz vorliegt.

7. Salz zur Verwendung nach einem der Ansprüche 1 bis 5, **dadurch gekennzeichnet, dass** es der folgenden Formel (II) entspricht:
A⁻, H₃N⁺-CH(R¹)-CH²-S-S-CH₂-CH(R₂)-CONH-CH(R₃)-COOR₄ (II)
in welcher:
∘ A⁻ ein Phosphat-, Chlorid-, Acetat-, Methansulfonat-, Borat-, Lactat-, Fumarat-, Succinat-, Hemisuccinat-, Citrat-, Tartrat-, Hemitartrat-, Maleat-, Ascorbat-, Hemifumarat-, Hexanoat-, Heptanoat-, Hippurat-, Hydrocinnamat-, Phenylglyoxylat- oder Nicotinatanion darstellt, wobei A⁻ vorzugsweise ein Fumaratanion darstellt;
∘ R₁ eine gesättigte oder ungesättigte, lineare oder verzweigte Kohlenwasserstoffkette mit 1 bis 6 Kohlenstoffatomen darstellt, substituiert oder nicht durch eine OR-, SR- oder S(O)R-Gruppe, in der R einen Wasserstoff, eine lineare oder verzweigte Kohlenwasserstoffkette aus 1 bis 4 Wasserstoffen, ein Phenyl- oder Benzylradikal darstellt;
∘ R₂ darstellt:
- eine Phenyl- oder Benzylgruppe, eventuell substituiert durch:
* 1 bis 5 Halogenatome, insbesondere Fluor, ein Hydroxyl oder ein Thiol, ein OR-Ether oder ein SR-Thioether, wobei R dieselbe Bedeutung hat wie oben,
* einen aromatischen oder heterozyklischen aromatischen Ring aus 5 oder 6 Atomen, wobei das Heteroatom ein Sauerstoff, ein Stickstoff oder ein Schwefel ist,
- eine Methylengruppe, substituiert durch einen aromatischen oder gesättigten Heterozyklus aus 5 oder 6 Atomen, wobei das Heteroatom ein Sauerstoff, ein Stickstoff oder ein Schwefel ist, wobei die Stickstoff- und Schwefelatome eventuell in Form von N-Oxid oder von S-Oxid oxidiert sind;
∘ R₃ darstellt:
- ein Wasserstoff,
- eine OH- oder OR-Gruppe, wobei R dieselbe Bedeutung hat wie oben,
- ein lineares oder verzweigtes C₁-C₆-Alkyl, eventuell substituiert durch eine OH-, OR-, SH- oder SR-Gruppe, wobei R dieselbe Bedeutung hat wie oben,
- eine Phenyl- oder Benzylgruppe, substituiert oder nicht durch 1 bis 5 Halogene, insbesondere Fluor, durch eine OR- oder SR-Gruppe, wobei R dieselbe Definition hat wie oben;
∘ OR₄ darstellt:
- ein Glyoclatradikal OCH₂COOR' oder Laktatradikal OCH (CH₃)COOR',
- eine Gruppe OCH₂OCOR' oder OCH(CH₃)OCOOR', oder
- ein Triclyceridradikal OCH(CH₂OCOR')₂ oder OCH₂-CH(OCOR')-CH₂OCOR', wobei R' ein lineares oder verzweigtes C₁-C₄-Alkyl darstellt.

8. Salz zur Verwendung nach Anspruch 1, **dadurch gekennzeichnet, dass** es aus den pharmazeutisch akzeptablen Salzen der folgenden Verbindungen ausgewählt ist:
1-(2-(1-(2,3-diacetoxypropoxycarbonyl)-ethylcarbamoyl)-3-thiophen-3-ylpropyldisulfanylmethyl)-3-methylsulfanylpropyl-amin,
1-(2-(1-(2-methansulfonylethoxycarbonyl)-ethylcarbamoyl)-3-thiophen-3-ylpropyldisulfanylmethyl)-3-methylsulfanylpropyl-amin,
1-(2-(1-(1-ethoxycarbonyloxyethoxycarbonyl))-ethylcarbamoyl)-3-thiophen-3-yl-propyldisulfanylmethyl)-3-methylsulfanylpropyl-amin,
1-(2-(1-ethoxycarbonylmethyloxycarbonylethylcarbamoyl)-3-thiophen-3-yl-propyldisulfanylmethyl)-3-methylsulfanylpropyl-amin,
1-(2-(1-(1-ethoxycarbonyloxyethoxycarbonyl)-2-hydroxypropylcarbamoyl)-3-thiophen-3-ylpropyldisulfanylmethyl)-3-methylsulfanylpropyl-amin,
1-(2-(1-(2-acetoxy-1-acetoxymethylethoxycarbonyl)-ethylcarbamoyl)-3-thiophen-3-ylpropyldisulfanylmethyl)-3-methylsulfanylpropyl-amin,
1-(2-(1-(2-hydroxy-1-hydroxymethylethoxycarbonyl)-ethylcarbamoyl)-3-thiophen-3-ylpropyldisulfanylmethyl)-3-methylsulfanylpropyl-amin,
1-(2-(1-(3,4,5,6-tetrahydroxytetrahydropyran-2-ylmethoxycarbonyl)-ethylcarbamoyl)-3-thiophen-3-yl-propyldisulfanylmethyl)-3-methylsulfanylpropyl-amin,
1-(2-(1-(1-ethoxycarbonyloxy-ethoxycarbonyl)-2-hydroxypropylcarbamoyl)-3-phenylpropyldisulfanylmethyl)-3-methylsulfanylpropyl-amin,
1-(2-(1-(2-acetoxy-1-acetoxymethyl-ethoxycarbonyl)-2-hydroxypropylcarbamoyl)-3-phenylpropyldisulfanylmethyl)-3-methylsulfanylpropyl-amin,
1-(2-((1-ethoxycarbonyloxy-ethoxycarbonylmethyl)-carbamoyl)-3-phenyl-propyldisulfanylmethyl)-3-methylsulfanylpropyl-amin,
3-(2-amino-4-methylsulfanyl-butyldisulfanyl)-2-benzyl-N-(5-ethyl-(1,3,4)-thiadiazol-2-yl)-propionamid,
1-(2-((1-ethoxycarbonyloxy-2-methyl-propoxycarbonylmethyl)-carbamoyl)-3-phenyl-propyldisulfanylmethyl)-3-methylsulfanyl-propyl-amin,
1-(2-((cyclohexyl-ethoxycarbonyloxy-methoxycarbonylmethyl)-carbamoyl)-3-phenyl-propyldisulfanylmethyl)-3-methylsulfanyl-propyl-amin,
1-(2-((ethoxycarbonyloxy-phenyl-methoxycarbonylmethyl)-carbamoyl)-3-phenyl-propyldisulfanylmethyl)-3-methylsulfanyl-propyl-amin,
3-methylsulfanyl-1-(3-phenyl-2-((1-propionyloxy-ethoxycarbonylmethyl)-carbamoyl)-propyldisulfanylmethyl)-propyl-amin,
1-(2-((2-methyl-1-propionyloxy-propoxycarbonylmethyl)-carbamoyl)-3-phenyl-propyldisulfanylmethyl)-3-methylsulfanyl-propyl-amin,
1-(2-((cyclohexyl-propionyloxy-methoxycarbonylmethyl)-carbamoyl)-3-phenyl-propyldisulfanylmethyl)-3-methylsulfanyl-propyl-amin,
3-methylsulfanyl-1-(3-phenyl-2-((phenyl-propionyloxy-methoxycarbonylmethyl)-carbamoyl)-propyldisulfanylmethyl)-propyl-amin.

9. Salz zur Verwendung nach Anspruch 1, **dadurch gekennzeichnet, dass** es sich um (5S, 10S)-10-Benzyl-16-methyl-11,14,18-trioxo-15,17,19-trioxa-2,7,8-trithia-12-azahenicosan-5-aminiumfumarat handelt.

10. Salz zur Verwendung nach einem der Ansprüche 1 bis 9, **dadurch gekennzeichnet, dass** eine therapeutische Dosis des Salzes dem Patienten bei Bedarf in einer bis vier Einnahmen verabreicht wird.

11. Salz zur Verwendung nach einem der Ansprüche 1 bis 10, **dadurch gekennzeichnet, dass** eine therapeutische Dosis des Salzes dem Patienten bei Bedarf zur Behandlung von Schmerzen verabreicht wird, die sich auf den Nervus trigeminus beziehen, vor allem bei Migränen oder Trigeminusneuralgien, wobei die Dosis zwischen 200 und 800 mg liegt.

12. Salz zur Verwendung nach einem der Ansprüche 1 bis 10, **dadurch gekennzeichnet, dass** das Salz zwecks Vorbeugung von migräneartigen Krisen verwendet wird.

13. Zusammensetzung zur Verwendung zwecks Vorbeugung oder Behandlung von Schmerzen, die sich auf den Nervus trigeminus beziehen, umfassend als Wirkstoff mindestens ein pharmazeutisch akzeptables Salz einer Verbindung der Formel (I) wie in einem der Ansprüche 1 bis 6, 8 und 9 definiert oder mindestens ein Salz der Formel (II) wie in Anspruch 7 definiert und mindestens einen pharmazeutisch akzeptablen Hilfsstoff.

14. Zusammensetzung zur Verwendung nach Anspruch 13, **dadurch gekennzeichnet, dass** sie ferner einen zweiten Wirkstoff umfasst, der zwecks Vorbeugung oder Behandlung der Schmerzen in Verbindung mit dem Nervus trigeminus nützlich ist.

15. Kit, umfassend:
i) eine erste Zusammensetzung, die mindestens ein pharmazeutisch akzeptables Salz einer Verbindung der Formel (I) wie oben definiert umfasst, und
ii) eine zweite Zusammensetzung, die mindestens einen zweiten Wirkstoff umfasst, der zur Behandlung des Schmerzes in Verbindung mit dem Nervus trigeminus nützlich ist, als Kombinationsprodukte für eine gleichzeitige, getrennte oder zeitlich gestaffelte Verwendung zwecks Vorbeugung oder Behandlung der Schmerzen in Verbindung mit dem Nervus trigeminus wie Migränen oder Trigeminusneuralgien.

16. Zusammensetzung nach Anspruch 14 oder Kit nach Anspruch 15 zur Verwendung zwecks Vorbeugung der Schmerzen in Verbindung mit dem Nervus trigeminus, **dadurch gekennzeichnet, dass** der zweite Wirkstoff aus den Betablockern, den Antidepressiva, den Anticonvulsiva, den Antagonisten der Calciumkanäle und/oder den Inhibitoren der Wirkung des CGRP ausgewählt ist, wobei er vorzugsweise aus dem Propranolol, dem Amitriptylin und/oder dem Topiramat ausgewählt ist.

17. Zusammensetzung nach Anspruch 14 oder Kit nach Anspruch 15 zur Verwendung zwecks Behandlung der Schmerzen in Verbindung mit dem Nervus trigeminus, **dadurch gekennzeichnet, dass** der zweite Wirkstoff aus den Analgetika, den nicht-steroiden Entzündungshemmern (AINS), den Opioiden, den Triptanen, den GABA-Modulatoren, den Inhibitoren der Natriumkanäle Nav 1.7, den Inhibitoren der Wirkung des CGRP und/oder den Cannabinoiden ausgewählt ist, wobei er vorzugsweise aus dem Morphin, dem Δ⁹ THC, dem Carbamazepin, dem Oxcarbazepin, dem Baclofen, dem Clonazepam, dem Lamotrigin, dem Gabapentin oder dem Pregabalin ausgewählt ist.

18. Verbindung nach einem der Ansprüche 1 bis 12 oder Zusammensetzung nach einem der Ansprüche 13, 14 oder 16 oder Kit nach einem der Ansprüche 15 oder 16 zur Verwendung zwecks Vorbeugung der Migränen oder der Trigeminusneuralgien.

19. Verbindung nach einem der Ansprüche 1 bis 12 oder Zusammensetzung nach einem der Ansprüche 13, 14 oder 17 oder Kit nach einem der Ansprüche 15 oder 18 zur Verwendung zwecks Behandlung der Migränen oder der Trigeminusneuralgien.

20. Verbindung nach einem der Ansprüche 1 bis 12 oder Zusammensetzung nach einem der Ansprüche 13, 14 oder 16 oder Kit nach einem der Ansprüche 15 oder 16 zur Verwendung zwecks Vorbeugung der Neuralgien im Wesentlichen des Trigeminus, der symptomatischen Neuralgien des Trigeminus, der Migränen, der cephalischen Hautallodynie, des Cluster-Kopfschmerzes des Gesichts oder der peripheren Trigeminusschmerzen in Verbindung mit der Multiplen Sklerose.

21. Verbindung nach einem der Ansprüche 1 bis 12 oder Zusammensetzung nach einem der Ansprüche 13, 14 oder 17 oder Kit nach einem der Ansprüche 15 oder 18 zur Verwendung zwecks Behandlung der Neuralgien im Wesentlichen des Trigeminus, der symptomatischen Neuralgien des Trigeminus, der Migränen, der cephalischen Hautallodynie, des Cluster-Kopfschmerzes des Gesichts oder der peripheren Trigeminusschmerzen in Verbindung mit der Multiplen Sklerose.

22. Zusammensetzung, umfassend 50 bis 800 mg eines Salzes wie in den Ansprüchen 1 bis 12 definiert, einen pharmazeutisch akzeptablen Hilfsstoff und einen zweiten Wirkstoff, wobei der zweite Wirkstoff zwecks Vorbeugung oder Behandlung der Schmerzen in Verbindung mit dem Nervus trigeminus nützlich ist.

## Claims

1. Pharmaceutically-acceptable salt, in particular acid addition salt, of a compound of formula (I), for use for the prevention or treatment of trigeminal nerve pain:
H₂N-CH(R₁)-CH₂-S-S-CH₂-CH(R₂)-CONH-CH(R₃)-COOR₄ (I)
wherein:
o R₁ represents:
- a saturated or unsaturated, linear or branched hydrocarbon chain, having 1 to 6 carbon atoms, optionally substituted by:
* an OR, SR or S(O)R radical, in each of these radicals, R represents a hydrogen, a linear or branched hydrocarbon chain of 1 to 4 carbon atoms, a phenyl or benzyl radical,
* a phenyl or benzyl radical,
- a phenyl or benzyl radical optionally substituted by:
* 1 to 5 halogens, notably fluorine,
* an OR, SR or S(O)R radical, in each of these radicals, R having the same meaning as previously,
- a methylene radical substituted by a 5 or 6 atom heterocycle, aromatic or saturated, having a nitrogen or sulfur atom as heteroatom, optionally oxidized in the form of N-oxide or S-oxide;
o R₂ represents:
- a phenyl or benzyl radical optionally substituted by:
* 1 to 5 halogens, notably fluorine,
* an OR or SR radical, in each of these radicals, R having the same meaning as previously,
* an amino group, optionally mono- or disubstituted by a cyclic or linear aliphatic group, of 1 to 6 carbon atoms,
* a 5 or 6 atom aromatic ring,
- a 5 or 6 atom aromatic heterocycle, the heteroatom being an oxygen, nitrogen or sulfur,
- a methylene group substituted by a 5 or 6 atom heterocycle, aromatic or saturated, the heteroatom being an oxygen, nitrogen or sulfur, the nitrogen and sulfur atoms can be oxidized in the form of N-oxide or S-oxide;
o R₃ represents:
- a hydrogen,
- an OH or OR group, R having the same meaning as previously,
- a saturated hydrocarbon (alkyl) chain, linear or branched, having 1 to 6 carbon atoms, optionally substituted by an OR or SR radical, in each of these radicals, R has the same meaning as previously,
- a phenyl or benzyl radical optionally substituted by:
* 1 to 5 halogens, notably fluorine,
* an OR or SR radical, R having the same meaning as previously, and
o OR₄ represents:
- a glycolate OCH₂COOR' or lactate OCH(CH₃)COOR' radical, in each of these radicals, R' represents:
• a linear or branched C₁-C₆ alkyl optionally substituted by a C₁-C₃ alkoxy group, preferably a C₁-C₄ alkyl group optionally substituted by a methoxy group,
• a C₅-C₈, cycloalkyl group, preferably a C₅-C₆ cycloalkyl group,
• a phenyl, benzyl, heteroaryl, alkyl heteroaryl group;
- an OCH(R")O(CO)OR' or OCH(R")O(CO)R' group, in each of these radicals, R' has the same meaning as previously and R" represents:
• a hydrogen,
• a linear or branched C₁-C₆ alkyl chain optionally substituted by a C₁-C₃ alkoxy group, preferably a C₁-C₄ alkyl group optionally substituted by a methoxy group,
• a C₅-C₈, cycloalkyl group, preferably a C₅-C₆ cycloalkyl group,
• a phenyl, benzyl, heteroaryl, alkyl heteroaryl group;
- an OCH(CH₂OCOR')₂ or OCH₂-CH(OCOR')-CH₂OCOR' triglyceride radical, in each of these radicals, R' having the same meaning as previously;
- a glycoside radical such as D-glucose, β-D-glucopyranose, α- or β-galactopyranose;
- a sulfonate OCH₂CH₂(SO₂)CH₃ radical;
- an OCH(CH₂OH)₂ radical.

2. Salt for use according to claim 1, **characterized in that** R₁ represents an alkyl radical having 1 to 4 carbon atoms substituted by an SR radical, R being as defined in claim 1 and representing, in particular, a linear or branched saturated hydrocarbon chain of 1 to 4 carbon atoms.

3. Salt for use according to claim 1 or 2, **characterized in that** R₂ represents a benzyl radical or methylene radical substituted by a 5 or 6 atom heterocycle, aromatic or saturated, having nitrogen or sulfur atom as heteroatom, optionally oxidized in the form of N-oxide or S-oxide.

4. Salt for use according to any one of the claims 1 to 3, **characterized in that** R₃ represents a hydrogen or an alkyl radical having 1 to 6 carbon atoms substituted by an OH or SH radical.

5. Salt for use according to any one of claims 1 to 4, **characterized in that** OR₄ represents an OCH (R")O(CO)OR' or OCH (R")O(CO)R' group, radical R' representing a C₁-C₄ alkyl chain, and radical R" representing a methyl, CH(CH₃)₂, cyclohexyl or phenyl radical.

6. Salt for use according to any one of claims 1 to 5, **characterized in that** the compound of formula (I) is in the form of a fumarate salt.

7. Salt for use according to any one of claims 1 to 5, **characterized in that** it conforms to the following formula (II):
A⁻, H₃N⁺-CH(R₁)-CH₂-S-S-CH₂-CH(R₂)-CONH-CH(R₃)-COOR₄ (II)
wherein:
o A⁻ represents a phosphate, chloride, acetate, methanesulfonate, borate, lactate, fumarate, succinate, hemisuccinate, citrate, tartrate, hemitartrate, maleate, ascorbate, hemifumarate, hexanoate, heptanoate, hippurate, hydrocinnamate, phenylglyoxylate or nicotinate anion; preferably A⁻ represents a fumarate anion;
o R₁ represents a saturated or unsaturated hydrocarbon chain, linear or branched, having 1 to 6 carbon atoms, substituted or not with an OR, SR or S(O)R group in which R represents a hydrogen, a linear or branched hydrocarbon chain of 1 to 4 carbons, a phenyl or benzyl radical;
o R₂ represents:
- a phenyl or benzyl group optionally substituted by:
* 1 to 5 halogens, notably fluorine, a hydroxyl or a thiol, an ether OR or a thioether SR, R having the same meaning as above,
* an aromatic ring or aromatic heterocycle with 5 or 6 atoms, the heteroatom being an oxygen, nitrogen or sulfur,
- a methylene group substituted by a 5 or 6 atom heterocycle, aromatic or saturated, the heteroatom being an oxygen, nitrogen or sulfur, the nitrogen or sulfur atoms optionally being oxidized in the form of N-oxide or S-oxide;
o R₃ represents:
- a hydrogen,
- an OH or OR group, R having the same meaning as above,
- a linear or branched C₁-C₆ alkyl, optionally substituted by an OH, OR, SH or SR group, R having the same meaning as above,
- a phenyl or benzyl group, substituted or not by 1 to 5 halogens, notably fluorine, by an OR or SR group, R having the same meaning as above;
o OR₄ represents:
- a glycolate OCH₂COOR' or lactate OCH(CH₃)COOR' radical;
- an OCH₂OCOR' or OCH(CH₃)OCOOR' group, or
- an OCH(CH₂OCOR')₂ or OCH₂-CH(OCOR')-CH₂OCOR' triglyceride radical,
R' representing a linear or branched C₁-C₄ alkyl.

8. Salt for use according to claim 1, **characterized in that** it is selected from the pharmaceutically-acceptable salts of the following compounds:
1-(2-(1-(2,3-diacetoxypropoxycarbonyl)-ethylcarbamoyl)-3-thiophen-3-ylpropyl disulfanylmethyl)-3-methylsulfanylpropyl-amine,
1-(2-(1-(2-methanesulfonylethoxycarbonyl)-ethylcarbamoyl)-3-thiophen-3-ylpropyl disulfanylmethyl)-3-methylsulfanylpropyl- amine,
1-(2-(1-(1-ethoxycarbonyloxyethoxycarbonyl))-ethylcarbamoyl)-3-thiophen-3-yl-propyl disulfanylmethyl)-3-methylsulfanylpropyl- amine,
1-(2-(1-ethoxycarbonylmethyloxycarbonylethylcarbamoyl)-3-thiophen-3-yl-propyl disulfanylmethyl)-3-methylsulfanylpropyl- amine,
1-(2-(1-(1-ethoxycarbonyloxyethoxycarbonyl)-2-hydroxypropylcarbamoyl)-3-thiophen-3-ylpropyldisulfanylmethyl)-3-methylsulfanylpropyl-amine,
1-(2-(1-(2-acetoxy-l-acetoxymethylethoxycarbonyl)-ethylcarbamoyl)-3-thiophen-3-yl propyldisulfanylmethyl)-3-methylsulfanylpropyl-amine,
1-(2-(1-(2-hydroxy-1-hydroxymethylethoxycarbonyl)-ethylcarbamoyl)-3-thiophen-3-yl propyldisulfanylmethyl)-3-methylsulfanylpropyl- amine,
1-(2-(1-(3,4,5,6-tetrahydroxytetrahydropyran-2-ylmethoxycarbonyl)-ethylcarbamoyl)-3-thiophen-3-yl-propyldisulfanylmethyl)-3-methylsulfanylpropyl-amine,
1-(2-(1-(1-ethoxycarbonyloxy-ethoxycarbonyl)-2-hydroxypropylcarbamoyl)-3-phenyl propyldisulfanylmethyl)-3-methylsulfanylpropyl-amine,
1-(2-(1-(2-acetoxy-1-acetoxymethyl-ethoxycarbonyl)-2-hydroxypropylcarbamoyl)-3-phenylpropyldisulfanylmethyl)-3-methylsulfanylpropyl-amine,
1-(2-((1-ethoxycarbonyloxy-ethoxycarbonylmethyl)-carbamoyl)-3-phenyl-propyl disulfanylmethyl)-3-methylsulfanylpropyl-amine,
3-(2-amino-4-methylsulfanyl-butyldisulfanyl)-2-benzyl-N-(5-ethyl-(1,3,4)-thiadiazol-2-yl)-propionamide,
1-(2-((1-ethoxycarbonyloxy-2-methyl-propoxycarbonylmethyl)-carbamoyl)-3-phenyl-propyldisulfanylmethyl)-3-methylsulfanyl-propyl-amine,
1-(2-((cyclohexyl-ethoxycarbonyloxy-methoxycarbonylmethyl)-carbamoyl)-3-phenyl-propyldisulfanylmethyl)-3-methylsulfanyl-propyl-amine,
1-(2-((ethoxycarbonyloxy-phenyl-methoxycarbonylmethyl)-carbamoyl)-3-phenyl-propyldisulfanylmethyl)-3-methylsulfanyl-propyl-amine,
3-methylsulfanyl-1-(3-phenyl-2-((1-propionyloxy-ethoxycarbonylmethyl)-carbamoyl)-propyldisulfanylmethyl)-propyl-amine,
1-(2-((2-methyl-1-propionyloxy-propoxycarbonylmethyl)-carbamoyl)-3-phenyl-propyldisulfanylmethyl)-3-methylsulfanyl-propyl-amine,
1-(2-((cyclohexyl-propionyloxy-methoxycarbonylmethyl)-carbamoyl)-3-phenyl-propyldisulfanylmethyl)-3-methylsulfanyl-propyl-amine,
3-methylsulfanyl-1-(3-phenyl-2-((phenyl-propionyloxy-methoxycarbonylmethyl)-carbamoyl)-propyl disulfanylmethyl)-propyl-amine.

9. Salt for use according to claim 1, **characterized in that** it is (5S, 10S)-10-benzyl-16-methyl-11,14,18-trioxo-15,17,19-trioxa-2,7,8-trithia-12-azahenicosan-5-aminium fumarate.

10. Salt for use according to any one claims 1 to 9, **characterized in that** a therapeutic dose of said salt is administered to the patient in need thereof in one to four administrations.

11. Salt for use according to any one of claims 1 to 10, **characterized in that** a therapeutic dose of said salt is administered to the patient in need thereof for treatment of trigeminal nerve pain, in particular migraines, or trigeminal neuralgia, said dose being comprised between 200 and 800 mg.

12. Salt for use according to any one of claims 1 to 10, **characterized in that** the salt is used for the prevention of migraine attacks.

13. Composition for use for prevention or treatment of trigeminal nerve pain, comprising as active ingredient at least one pharmaceutically-acceptable salt of a compound of formula (I) such as defined in any one of claims 1 to 6, 8 and 9 or at least one salt of formula (II) such as defined in claim 7, and at least one pharmaceutically-acceptable excipient.

14. Composition for use according to claim 13, **characterized in that** it further comprises a second active ingredient useful to prevent or treat trigeminal nerve pain.

15. Kit comprising:
i) a first composition comprising at least one pharmaceutically-acceptable salt of a compound of formula (I) such as defined previously, and
ii) a second composition comprising at least a second active ingredient useful to treat trigeminal nerve pain as combination products for simultaneous, separate or time-staggered use in the prevention or treatment of trigeminal nerve pain, such as migraines or trigeminal neuralgia.

16. Composition according to claim 14 or kit according to claim 15 for use for the prevention of trigeminal nerve pain, **characterized in that** the second active ingredient is selected from beta blockers, antidepressants, anticonvulsants, calcium channel blockers and/or CGRP inhibitors, preferably it is selected from propranolol, amitriptyline and/or topiramate.

17. Composition according to claim 14 or kit according to claim 15 for use for the treatment of trigeminal nerve pain, **characterized in that** the second active ingredient is selected from analgesics, nonsteroidal antiinflammatory drugs (NSAIDs), opioids, triptans, GABA modulators, Nav 1.7 sodium channel blockers, CGRP inhibitors and/or cannabinoids, preferably it is selected from morphine, Δ⁹ THC, carbamazepine, oxcarbazepine, baclofen, clonazepam, lamotrigine, gabapentin or pregabalin.

18. Compound according to any one of claims 1 to 12, or composition according to any one of claims 13, 14, or 16, or kit according to any one of claims 15 or 16, for use to prevent migraines or trigeminal neuralgia.

19. Compound according to any one of claims 1 to 12, or composition according to any one of claims 13, 14, or 17, or kit according to any one of claims 15 or 18, for use to treat migraines or trigeminal neuralgia.

20. Compound according to any one of claims 1 to 12, or composition according to any one of claims 13, 14, or 16, or kit according to any one of claims 15 or 16, for use to prevent essential trigeminal neuralgia, symptomatic trigeminal neuralgia, migraines, cephalic cutaneous allodynia, cluster headaches, or peripheral trigeminal pain associated with multiple sclerosis.

21. Compound according to any one of claims 1 to 12, or composition according to any one of claims 13, 14, or 17, or kit according to any one of claims 15 or 18, for use to treat essential trigeminal neuralgia, symptomatic trigeminal neuralgia, migraines, cephalic cutaneous allodynia, cluster headaches, or peripheral trigeminal pain associated with multiple sclerosis.

22. Composition comprising 50 to 800 mg of a salt such as defined in claims 1 to 12, a pharmaceutically-acceptable excipient and a second active ingredient, said second active ingredient being useful to prevent or treat trigeminal nerve pain.
